(19) Europäisches Patentamt
European Patent Office
Office européen des brevets

(11) Publication number: **0 003 008 A1**

# (12) EUROPEAN PATENT APPLICATION

(21) Application number: **78850029.6**

(22) Date of filing: **19.12.78**

(51) Int. Cl.²: **C 07 F 9/40,** A 61 K 31/66

(30) Priority: **22.12.77 GB 5357877**
**22.12.77 GB 5357977**

(43) Date of publication of application: **11.07.79**
**Bulletin 79/14**

(84) Designated Contracting States: **BE CH DE FR GB IT LU NL SE**

(71) Applicant: **Astra Läkemedel Aktiebolag, Strängnäsvägen 44, S-151 85 Södertälje (SE)**

(72) Inventor: **Helgstrand, Ake John Erik, Bäverstigen 32, S-150 23 Enhörna (SE)**
Inventor: **Johansson, Karl Nils-Gunnar, Bäverstigen 19, S-150 23 Enhörna (SE)**
Inventor: **Misiorny, Alfons, Lerbäcksgränd 30, S-124 43 Bandhagen (SE)**
Inventor: **Norén, Jan-Olof, Varstavägen 77, S-140 32 Grödinge (SE)**
Inventor: **Stening, Göran Bertil, Puckvägen 8, S-151 50 Södertälje (SE)**

(74) Representative: **Wurm, Bengt R. et al, Kvarnbargagatan 16, S-151 85 Södertälje (SE)**

## (54) Amide derivatives of phosphonoformic acid, pharmaceutical compositions and methods for combating virus infections.

(57) A pharmaceutical preparation containing as active ingredient a compound of the formula

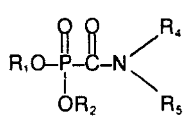

I

wherein $R_1$ and $R_2$, which are the same or different, each is selected from the group consisting of hydrogen, alkyl groups containing 1–6 carbon atoms; cycloalkyl groups containing 3–6 carbon atoms; cycloalkyl-alkyl groups containing 4–6 carbon atoms; phenyl; benzyl, and wherein $R_4$ and $R_5$ are the same or different, and each are selected from the group consisting of hydrogen, alkyl groups containing 1–6 carbon atoms, cycloalkyl groups containing 4–6 carbon atoms, phenyl, and benzyl; or $R_4$ and $R_5$ together with the nitrogen atom may form a pyrrolidine or piperidine ring; or a physiologically acceptable salt or an optical isomer thereof; novel compounds within formula I, methods for their preparation and their medicinal use.

EP 0 003 008 A1

0003008

ASTRA LÄKEMEDEL AKTIEBOLAG
Södertälje/SWEDEN

Inventors: E Helgstrand, N G Johansson, A Misiorny, J O Norén, G Stening

LA 590-1
78-12-12
IH/LB

Amide derivatives of phosphonoformic acid, pharmaceutical compositions and methods for combatting virus infections.

FIELD OF THE INVENTION

The present invention relates to novel pharmaceutical compositions and to a novel method for selectively combatting viruses, such as herpes viruses, influenza viruses, RNA tumor viruses, etc., which can cause various diseases in animals including man. Such diseases include both common infections and neoplastic diseases, i.e. cancer. In those cases where, the active ingredient in the composition is a novel compound, the invention also comprises the novel compounds *per se*.

BACKGROUND OF THE INVENTION

The effects of viruses on bodily functions is the end result of changes occurring at the cellular and subcellular levels.

The pathogenic changes at the cellular level are different for different combinations of viruses and host cells. While some viruses cause a general destruction (killing) of certain cells, other may transform cells to a neoplastic state.

Important common viral infections are herpes dermatitis (including herpes labialis), herpes keratitis, herpes genitalis, herpes zoster, herpes encephalitis, infectious mononucleosis and cytomegalovirus infections all of which are caused by viruses belonging to the herpesvirus group. Other important viral diseases are influenza A and B which are caused by influenza A and B virus respectively. Another important common viral disease is viral hepatitis and especially hepatitis B virus infections are widely spread. Effective and selective antiviral agents are needed for the treatment of these diseases.

Several different viruses of both DNA and RNA type have been shown to cause tumors in animals. The effect of cancerogenic chemicals can on animals result in activation of latent tumor viruses. It is possible that tumor viruses are involved in human tumors. The most likely human cases known today are leucemias, sarcomas, breast carcinomas, Burkitt lymphomas, nasopharyngeal carcinomas and cervical cancers where RNA tumor viruses and herpes viruses are indicated. This makes the search for selective inhibitors of tumorogenic viruses and their functions an important undertaking in the efforts to treat cancer.

A most important common feature of the interaction between viruses and cells is the replication or transcription of the specific viral genetic information carried by viral nucleic acids. These viral nucleic acids are of two kinds, deoxyribonucleic acids (DNA) or ribonucleic acids (RNA). The primary genetic information of the cell is carried by cell DNA. DNA and RNA synthesis involves complex enzymes called DNA and RNA polymerases respectively. The genetic information is transferred to the new nucleic acid from a template nucleic acid. There are four general ways in which these nucleic acids can

be replicated or transcribed

1. DNA (template) $\xrightarrow[\text{DNA polymerase}]{\text{DNA-dependent}}$ DNA

2. RNA (template) $\xrightarrow[\text{RNA polymerase}]{\text{RNA-dependent}}$ RNA

3. DNA (template) $\xrightarrow[\text{RNA polymerase}]{\text{DNA-dependent}}$ RNA

4. RNA (template) $\xrightarrow[\text{DNA polymerase}]{\text{RNA-dependent}}$ DNA
(reverse transcriptase)

Processes 1 and 3 are used by cells. DNA viruses such as herpesviruses also use process 1 but the enzyme is different from that of the cell. RNA viruses such as influenza virus use process 2 and the RNA tumor viruses (retroviruses) can transcribe its RNA to DNA according to process 4.

The viral polymerases and the viral nucleic acid syntheses are essential not only for ordinary (productive) virus infections but also for viral transformation of cells to a neoplastic state leading to cancer (tumorogenic function of virus). In the latter case DNA produced by DNA viruses such as herpesvirus or transcribed from RNA of RNA tumor viruses and which carries the genetic information for cell transformation can be integrated into the host cell DNA. This integration, or later acts as a consequence of integration (such as interaction with cancerogenic chemicals), can then lead to the transformation of the host cell. The implications of inhibiting reverse transcriptase for cell transformation are also described in U.S. patent 3,979,511.

Since the viral polymerases in most cases differ from the cellular ones these viral enzymes and viral nucleic acid syntheses are good targets for specific antiviral chemotherapy including chemotherapy of cancer caused by viruses. It should be noted that many compounds presently used for chemotherapy of cancer are inhibitors of nucleic acid synthesis. It is therefore possible that antiviral compounds which are also inhibitors of nucleic acid synthesis can affect

tumor cells directly. There is a need for an effective antiviral agent preferably having a selective inhibiting effect on a specific viral function of the virus to be combatted. It is, therefore, a general object of the present invention to provide a novel method for combatting virus infections using an antiviral agent which exerts a selective inhibiting effect on viral functions but which exerts only a negligible inhibiting effect on functions of the host cells.

THE INVENTION

It has been found according to the present invention that the compounds of the formula

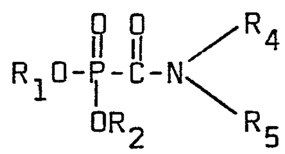

I

wherein $R_1$ and $R_2$, which are the same or different, each is selected from the group consisting of hydrogen, alkyl groups containing 1-6 carbon atoms; cycloalkyl groups containing 3-6 carbon atoms; cycloalkyl-alkyl groups containing 4-6 carbon atoms; phenyl; and benzyl, and wherein $R_4$ and $R_5$ are the same or different, and each are selected from the group consisting of hydrogen, alkyl groups containing 1-6 carbon atoms, cycloalkyl groups containing 4-6 carbon atoms, phenyl, and benzyl; or $R_4$ and $R_5$ together with the nitrogen atom may form a pyrrolidine or piperidine ring; and physiologically acceptable salts thereof, inhibit certain viral functions including tumorogenic functions and the multiplication of viruses.

It is understood that the reference to "physiologically acceptable salts" of the compounds of the formula I in the present specification and claims relates only to such compounds which can form salts. Compounds wherein at least one of $R_1$ and $R_2$ is hydrogen can form salts. Compounds wherein both $R_1$ and $R_2$ are different from hydrogen do not form salts.

Since the compounds of the formula I, when $R_1$ and $R_2$ are different, contain an asymmetric center, they exist in the form of optically active forms, and can be resolved into their optical antipodes by known methods.

In this specification, the compounds of the invention are named as derivatives of the compound hydroxycarbonylphosphonic acid, which compound also is known under the name phosphonoformic acid.

The compounds of the formula I and physiologically acceptable salts thereof are useful in therapeutic and/or prophylactic treatment of viral diseases and may be useful in therapeutic and/or prophylactic treatment of cancer caused by viruses.

PRIOR ART

Various carbamoylphosphonates are described in for example US Patent Nos. 2 909 558, 3 849 102, 3 929 448, 3 952 074, 3 997 544 and in Chemical Abstracts Vol. 50, 793 (1956)and Vol. 45, 542 (1951). However, these compounds have not been suggested for any pharmacological use.

DETAILED DESCRIPTION OF INVENTION

The present invention provides

A. A method for the treatment of diseases caused by viruses in animals including man, comprising administering to an animal so infected a therapeutically effective amount of a compound of the formula I or a physiologically acceptable salt thereof.

B. A method for the treatment of virus-induced neoplastic diseases in animals including man, by inhibiting the transformation of virus-infected cells, characterized by administering to an animal so infected a therapeutically effective amount of a compound of the formula I or a physiologically acceptable salt thereof.

C. A method for the treatment of diseases caused by viruses in animals including man, by inhibiting the activity of viral polymerase, characterized by administering to an animal so infected a compound of the formula I or a physiologically acceptable salt thereof in an amount effective for inhibiting the activity of said viral polymerase.

D. A method for inhibiting the activity of reverse transcriptases of viruses in animals including man, by administration to an animal a compound of the formula I or a physiologically acceptable salt thereof in an amount sufficient for inhibiting the activity of said reverse transcriptase. Particular reverse transcriptases are the reverse transcriptases of retroviruses, such as visna, sarcoma and leucemia viruses.

E. A method for inhibiting the multiplication of virus, in particular herpesviruses, influenza virus and hepatitis B virus, and retroviruses in animals including man, by administering to an animal in need of such treatment a compound of the formula I or a physiologically acceptable salt thereof in an amount sufficient for inhibiting said multiplication.

F. A method for inhibiting the growth of virus-transformed cells in animals including man, characterized by administering to an animal in need of such treatment a compound of the formula I or a physiologically acceptable salt thereof in an amount sufficient for inhibiting said growth.

G. A method for the treatment of virus-induced neoplastic diseases in animals including man, by inhibiting the multiplication of tumor viruses, characterized by administering to an animal in need of such treatment a compound of the formula I or a physiologically acceptable salt thereof in an amount sufficient for inhibiting such multiplication.

H. A method for the treatment of virusinduced neoplastic diseases in animals including man by inhibiting the activity of reverse transcriptase, characterized by administering to an animal so infected a compound of the formula I or a physiologically acceptable salt thereof in an amount effective for inhibiting the activity of said reverse transcriptase.

I. A method for the treatment of neoplastic diseases in animals including man, characterized by administering to an animal a therapeutically effective amount of a compound of the formula I or a physiologically acceptable salt thereof.

The invention also relates to the use of a compound of the formula I or a physiologically acceptable salt thereof, in each of the above given methods A, B, C, D, E, F, G, H, and I. For example, the invention relates to the use of a compound of the formula I or a physiologically acceptable salt thereof, for

(a) inhibiting the replication of virus in animals including man, in particular herpesvirus, influenza virus and hepatitis B viruses; and

(b) for inhibiting the growth of virus-transformed cells in animals including man.

Furthermore, the invention provides pharmaceutical preparations comprising as active ingredient a compound of the formula I or a physiologically acceptable salt thereof, optionally in association with a pharmaceutically acceptable carrier. The invention also encompasses a process for the preparation of a medicine having antiviral activity, characterized in that a compound of the formula I or a physiologically acceptable salt thereof is brought into an administration form suitable for therapeutical purposes, and the shaped medicine obtained by such process.

Most of the compounds within the formula I are novel compounds, and in those cases where the active ingredient in the compositions is such a compound, the invention also comprises the novel compounds per se.

Specifically, compounds included in formula I, and physiologically acceptable salts thereof, wherein $R_1$, $R_2$, $R_4$ and $R_5$ are combined as follows are not disclosed (specifically or generically) in the prior art:

a) Compounds wherein both or $R_1$ and $R_2$ are hydrogen;
b) Compounds wherein $R_1$ is hydrogen and $R_2$ is phenyl; benzyl, cycloalkyl, or cycloalkyl-alkyl;
c) Compounds wherein at least one of the groups $R_1$, $R_2$, $R_4$ and $R_5$ are cycloalkyl or cycloalkyl-alkyl;
d) Compounds wherein at least one of the groups $R_4$ and $R_5$ is benzyl;

In tables below, compounds of the formula I which are known from the prior art are indicated. The invention includes within its scope those compounds per se which are included in formula I and which are not known in the prior art. Furthermore, groups of novel compounds, which also are included in the scope of the invention, are indicated in the preferred groups of radicals which are enumerated elsewhere in this specification. Those individual compounds which are enumerated in tables below, or which are exemplified in working examples, and which are not indicated as known in the art, are believed to be novel and are included within the scope of the invention.

The compounds of the formula I may be hydrolyzed in vivo to give phosphonoformic acid or ionized forms thereof, which are antiviral agents. In a more generalized aspect the invention includes within its scope the use of all physiologically acceptable compounds (including physiologically acceptable salts thereof) of the formula I, wherein $R_1$, $R_2$, $R_4$ and $R_5$ are any pharmaceutically acceptable organic group, which by in vivo hydrolysis is capable of forming phosphonoformic acid or a physiologically acceptable salt thereof in the animal body (i.e. bioprecursors to phosphonoformic acid) for the treatment of virus infections and related ailments, as previously described, in animals including man, and pharmaceutical compositions containing such compounds.

Phosphonoformic acid and physiologically acceptable salts thereof inhibit viral functions such as polymerases including reverse transcriptase and virus multiplication and have effects on virus infections and virus-related tumors in animal models. The antiviral effects of trisodium phosphonoformate is described by Helgstrand et.al. Science 201, 819 (1978).

An important aspect of the invention is that the radicals $R_1$, $R_2$, $R_4$ and $R_5$ in formula I can be chosen in such a way that the compounds of formula I and physiologically acceptable salts thereof possess more favourable pharmacokinetic properties than phosphonoformic acid and physiologically acceptable salts thereof. Such favourable pharmacokinetic properties include better tissue penetration, better oral absorption and prolonged activity.

Although the present invention relates broadly to a novel method for selectively combating viral diseases in animals and man, and pharmaceutical preparations to be used in such treatment, it will be particularly useful in the treatment of herpesvirus infections, influenza virus infections, hepatitis B virus infections and cancer caused by herpesviruses and RNA tumor viruses.

An especially important area of use for the compositions of the present invention is in the treatment of herpesvirus infections. Among the herpesviruses may be mentioned Herpes simplex type 1 and 2, varicella (Herpes zoster), virus causing infectious mononucleosis (i.e. Epstein-Barr virus), and cytomegalovirus. Important diseases caused by herpesviruses are herpes dermatitis, (including herpes labialis), herpes genitalis, herpes keratitis and herpes encephalitis. Another important area of use for the compositions of the present invention is in the treatment of infections caused by orthomyxoviruses, i.e. influenza viruses of type A and type B. A further area of use is the treatment of infections caused by viruses such as hepatitis virus A and hepatitis virus B, papillomaviruses, adenoviruses and poxviruses.

Other possible areas of use for the compositions of the present invention are in the treatment of infections caused by picornaviruses, togaviruses including arboviruses, retroviruses (e.g. leucoviruses), arenaviruses, coronaviruses, rhabdoviruses, paramyxoviruses, hepatitis non A and B virus, iridoviruses, papovaviruses, parvoviruses, reoviruses, and bunyaviruses.

Another possible area of use for the compositions of the present invention are in the treatment of cancer and tumors, particularly those caused by viruses. This effect may be obtained in different ways, i.e. by inhibiting the trans-formation of virus-infected cells to a neoplastic state, by inhibiting the spread of viruses from transformed cells

to other normal cells and by arresting the growth of virus transformed cells. A particular area of use for the compositions of the present invention is in the inhibition of reverse transcriptases of RNA tumor viruses. The viruses in this group include all of the transforming sarcoma C-type viruses, the leucemia C-type viruses and the mammary B-type viruses. Possible areas of use for the compositions of the present invention with respect to cancer chemotherapy are treatment of leucemias, lymphomas including Burkitt lymphomas and Hodgkin's disease, sarcomas, breast carcinoma, nasopharyngeal carcinomas and cervical cancers in which RNA tumor viruses and herpesviruses are indicated. Other possible areas of use for the composition of the present invention with respect to cancer chemotherapy are treatment of multiple myeloma and cancer of the lungs (and bronchus), the stomach, the liver, the colon, the bladder, the lips, the bones, the kidneys, the ovary, the prostate, the pancreas, the skin (melanoma), the rectum, the salivary glands, the mouth, the esophagus, the testis, the brain (and cranial meninges), the thyroid gland, the gallbladder (and ducts), the nose, the larynx, connective tissues, the penis, the vulvas, the vagina, the corpus uteri, the tongue, the breasts, and the cervix.

Illustrative examples of the meanings of the radicals $R_1$, $R_2$, $R_4$ and $R_5$ are:

alkyl: $-CH_3$, $-C_2H_5$, n-$C_3H_7$, i-$C_3H_7$, n-$C_4H_9$, 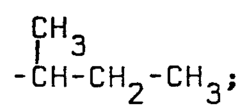;

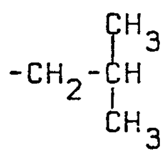, $-C(CH_3)_3$, $-(CH_2)_4CH_3$, 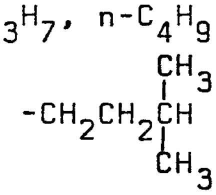, $-(CH_2)_4CH_3$,

$-(CH_2)_5CH_3$, $-(CH_2)_2CH(CH_3)CH_2CH_3$

cycloalkyl: 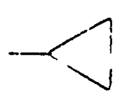 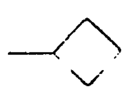 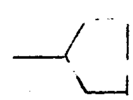 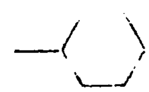

cycloalkylalkyl: $-CH_2$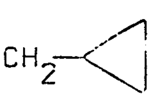 $-CH_2$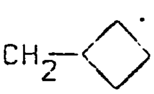 $-CH_2$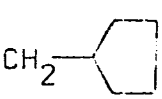

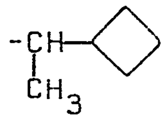

The above illustrative examples are intended to illustrate the meanings of all the radicals $R_1$, $R_2$, $R_4$ and $R_5$ within the boundaries with regard to number of carbon atoms which are prescribed for each radical.

Preferred groups of the radicals $R_1$ and $R_2$ are:

1. the group consisting of straight and branched alkyl groups containing 1-6 carbon atoms, phenyl, and benzyl,

2. the group consisting of straight and branched alkyl groups containing 1-4 carbon atoms, phenyl, and benzyl,

3. the group consisting of straight and branched alkyl groups containing 1-6 carbon atoms,

4. the group consisting of straight alkyl groups containing 1-4 carbon atoms, that is methyl, ethyl, n-propyl and butyl,

5. phenyl,

6. benzyl,

7. hydrogen. (novel compounds)

It is especially preferred that $R_1$ and $R_2$ are the same.

Preferred groups of the radicals $R_4$ and $R_5$ are:

8. $R_4$ and $R_5$ are the same or different and are each selected from hydrogen, alkyl groups containing 1-4 carbon atoms, phenyl and benzyl,

9. $R_4$ and $R_5$ are the same or different and are each selected from hydrogen and alkyl groups containing 1-4 carbon atoms,

10. $R_4$ and $R_5$ are the same or different and are each selected from hydrogen and phenyl,

11. $R_4$ and $R_5$ are the same or different and are each selected from hydrogen and benzyl,

12. $R_4$ and $R_5$ are both hydrogen,

13. $R_4$ is hydrogen and $R_5$ is selected from alkyl groups containing 1-4 carbon atoms,

14. $R_4$ is hydrogen and $R_5$ is benzyl,

15. $R_4$ is hydrogen and $R_5$ is selected from cycloalkyl groups with 4-6 carbon atoms.

Preferred combinations of $R_1$ & $R_2$ and $R_4$ & $R_5$ are:

1. $R_1$ and $R_2$ are the same and are selected from straight or branched alkyl groups containing 1-6 carbon atoms, phenyl and benzyl, and $R_4$ and $R_5$ are as defined in anyone of the groups (8) - (15) above,

2. $R_1$ and $R_2$ are the same and are selected from straight and branched alkyl groups containing 1-4 carbon atoms, phenyl and benzyl, and $R_4$ and $R_5$ are as defined in anyone of the groups (8) - (15) above,

3. $R_1$ and $R_2$ are the same and are selected from straight and branched alkyl groups containing 1-6 carbon atoms, and $R_4$ and $R_5$ are as defined in anyone of the groups (8) - (15) above,

4. $R_1$ and $R_2$ are phenyl, and $R_4$ and $R_5$ are as defined in anyone of the groups (8) - (15) above,

5. $R_1$ and $R_2$ are benzyl, and $R_4$ and $R_5$ are as defined in anyone of the groups (8) - (15) above, (novel compounds)

6. $R_1$ and $R_2$ are hydrogen, and $R_4$ and $R_5$ are as defined in any one of the groups (8) - (15) above.

In the above six preferred combinations of $R_1$ & $R_2$ and $R_4$ & $R_5$ it is preferred that $R_4$ and $R_5$ are same or different and are each selected from hydrogen, alkyl groups containing 1-4 carbon atoms, phenyl and benzyl.

Examples of preferred compounds of the invention are given in the following table. In the right margin it is indicated whether the compound has been specifically disclosed in the prior art; all the other compounds are believed to be novel, and thus constitute a further aspect of the invention;

| $R_1$ | $R_2$ | $R_4$ | $R_5$ | |
|---|---|---|---|---|
| $CH_3$ | $CH_3$ | $CH_3$ | H | Prior art |
| $CH_3$ | $CH_3$ | $C_2H_5$ | H | |
| $CH_3$ | $CH_3$ | n-$C_3H_7$ | H | |
| $CH_3$ | $CH_3$ | i-$C_3H_7$ | H | |
| $CH_3$ | $CH_3$ | n-$C_4H_9$ | H | |
| $CH_3$ | $CH_3$ | H | H | Prior art |
| $CH_3$ | $CH_3$ | $CH_3$ | $CH_3$ | |
| $CH_3$ | $CH_3$ | $C_2H_5$ | $C_2H_5$ | |
| $CH_3$ | $CH_3$ | i-$C_3H_7$ | i-$C_3H_7$ | |
| $CH_3$ | $CH_3$ | phenyl | H | |

| $R_1$ | $R_2$ | $R_4$ | $R_5$ | |
|---|---|---|---|---|
| $CH_3$ | $CH_3$ | benzyl | H | |
| $C_2H_5$ | $C_2H_5$ | $CH_3$ | H | Prior art |
| $C_2H_5$ | $C_2H_5$ | $C_2H_5$ | H | Prior art |
| $C_2H_5$ | $C_2H_5$ | $n-C_3H_7$ | H | Prior art |
| $C_2H_5$ | $C_2H_5$ | $i-C_3H_7$ | H | Prior art |
| $C_2H_5$ | $C_2H_5$ | $n-C_4H_9$ | H | |
| $C_2H_5$ | $C_2H_5$ | H | H | |
| $C_2H_5$ | $C_2H_5$ | $i-C_4H_9$ | H | |
| $C_2H_5$ | $C_2H_5$ | $C_2H_5$ | $C_2H_5$ | Prior art |
| $C_2H_5$ | $C_2H_5$ | $i-C_3H_7$ | $i-C_3H_7$ | |
| $C_2H_5$ | $C_2H_5$ | $n-C_3H_7$ | $n-C_3H_7$ | |
| $C_2H_5$ | $C_2H_5$ | $n-C_4H_9$ | $n-C_4H_9$ | Prior art |
| $C_2H_5$ | $C_2H_5$ | $i-C_4H_9$ | $i-C_4H_9$ | Prior art |
| $C_2H_5$ | $C_2H_5$ | phenyl | phenyl | |
| $C_2H_5$ | $C_2H_5$ | benzyl | benzyl | |
| $n-C_3H_7$ | $n-C_3H_7$ | $CH_3$ | H | |
| $n-C_3H_7$ | $n-C_3H_7$ | $C_2H_5$ | H | |
| $n-C_3H_7$ | $n-C_3H_7$ | $n-C_3H_7$ | H | |
| $n-C_3H_7$ | $n-C_3H_7$ | $i-C_3H_7$ | H | |
| $n-C_3H_7$ | $n-C_3H_7$ | $n-C_4H_9$ | H | |
| $n-C_3H_7$ | $n-C_3H_7$ | H | H | Prior art |
| $n-C_3H_7$ | $n-C_3H_7$ | $C_2H_5$ | $C_2H_5$ | |
| $i-C_3H_7$ | $i-C_3H_7$ | $C_2H_5$ | H | |
| $n-C_4H_9$ | $n-C_4H_9$ | $CH_3$ | H | |
| $n-C_4H_9$ | $n-C_4H_9$ | $C_2H_5$ | H | |
| $n-C_4H_9$ | $n-C_4H_9$ | $n-C_3H_7$ | H | |

| $R_1$ | $R_2$ | $R_4$ | $R_5$ |
|---|---|---|---|
| n-$C_4H_9$ | n-$C_4H_9$ | i-$C_3H_7$ | H |
| n-$C_4H_9$ | n-$C_4H_9$ | n-$C_4H_9$ | H |
| n-$C_4H_9$ | n-$C_4H_9$ | $C_2H_5$ | $C_2H_5$ |
| n-$C_4H_9$ | n-$C_4H_9$ | H | H Prior art |
| n-$C_4H_9$ | n-$C_4H_9$ | benzyl | H |
| n-$C_5H_{11}$ | n-$C_5H_{11}$ | $CH_3$ | H |
| n-$C_5H_{11}$ | n-$C_5H_{11}$ | $C_2H_5$ | H |
| n-$C_5H_{11}$ | n-$C_5H_{11}$ | n-$C_3H_7$ | H |
| n-$C_5H_{11}$ | n-$C_5H_{11}$ | phenyl | H |
| n-$C_6H_{13}$ | n-$C_6H_{13}$ | $CH_3$ | H |
| n-$C_6H_{13}$ | n-$C_6H_{13}$ | $C_2H_5$ | H |
| n-$C_6H_{13}$ | n-$C_6H_{13}$ | $CH_3$ | $CH_3$ |
| n-$C_6H_{13}$ | n-$C_6H_{13}$ | $C_2H_5$ | $C_2H_5$ |
| n-$C_6H_{13}$ | n-$C_6H_{13}$ | H | H |
| n-$C_6H_{13}$ | n-$C_6H_{13}$ | i-$C_3H_7$ | i-$C_3H_7$ |
| phenyl | phenyl | $CH_3$ | H |
| phenyl | phenyl | $C_2H_5$ | H |
| phenyl | phenyl | phenyl | H |
| phenyl | phenyl | phenyl | H |
| benzyl | benzyl | $CH_3$ | H |
| benzyl | benzyl | $C_2H_5$ | H |
| benzyl | benzyl | H | H |
| benzyl | benzyl | $C_2H_5$ | $C_2H_5$ |

| $R_1$ | $R_2$ | $R_4$ | $R_5$ |
|---|---|---|---|
| H | H | H | H |
| H | H | H | $CH_3$ |
| H | H | H | $C_2H_5$ |
| H | H | H | $n-C_3H_7$ |
| H | H | H | $i-C_3H_7$ |
| H | H | H | $n-C_4H_9$ |
| H | H | H | 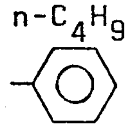 |
| H | H | H | $-CH_2-$ 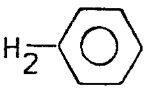 |
| H | H | H | 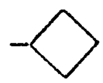 |
| H | H | H | 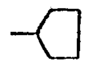 |
| H | H | H | 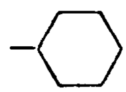 |
| H | H | $CH_3$ | $CH_3$ |
| H | H | $C_2H_5$ | $C_2H_5$ |
| H | H | $n-C_3H_7$ | $n-C_3H_7$ |
| H | H | $i-C_3H_7$ | $i-C_3H_7$ |
| H | H | $n-C_4H_9$ | $n-C_4H_9$ |
| H | H | 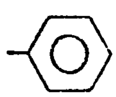 | 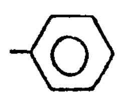 |
| H | H | $-CH_2-$ 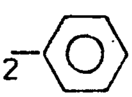 | $-CH_2-$ 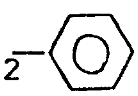 |
| H | H | $-NR_4R_5$ = - 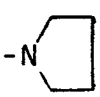 | |
| H | H | $-NR_4R_5$ = - 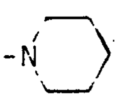 | |

Particularly preferred compounds are:

| $R_1$ | $R_2$ | $R_4$ | $R_5$ | Code |
|---|---|---|---|---|
| $C_2H_5$ | $C_2H_5$ | H | H | VIS 203 prior art |
| $C_2H_5$ | $C_2H_5$ | H | $C_2H_5$ | VIS 225 prior art |
| $C_2H_5$ | $C_2H_5$ | H | benzyl | VIS 229 |
| H | $C_2H_5$ | H | benzyl | VIS 444 |
| H | H | H | $C_2H_5$ | VIS 230 |
| H | H | $C_2H_5$ | $C_2H_5$ | VIS 232 |
| H | H | H | benzyl | VIS 231 |
| phenyl | phenyl | H | benzyl | VIS 445 |
| $C_2H_5$ | $C_2H_5$ | $C_2H_5$ | $C_2H_5$ | VIS 228 prior art |

and physiologically acceptable salts of these compounds.

Salts of the active substances

Physiologically acceptable salts of those active substances of the formula I which form salts are prepared by methods known in the art as illustrated in the following. Metal salts can be prepared by reacting a metal hydroxide with the active substances in acid form. Examples of metal salts of the active substances which can be prepared in this way are salts containing Li, Na, K, Ca, Mg, Zn, Mn and Ba. A less soluble metal salt can be precipitated from a solution of a more soluble salt by addition of a suitable metal compound. Thus for examples, Ca, Ba, Zn, Mg and Mn salts of the active substances can be prepared from sodium salts thereof. The metal ion of a metal salt of the active substances can be exchanged by hydrogen ions, other metal ions, ammonium ion and ammonium ions substituted by one or more organic radicals by using a cation exchanger as shown

in the following examples.

Examples of other useful salts which can be prepared in this way are the salts of the formula

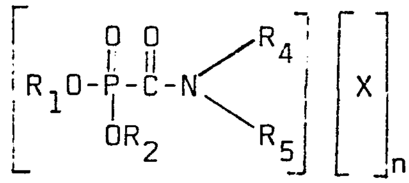

in which formula n is 1 or 2 and X is a salt-forming component such as $NH_3$, $CH_3NH_2$, $C_2H_5NH_2$, $C_3H_7NH_2$, $C_4H_9NH_2$, $C_5H_{11}NH_2$, $C_6H_{13}NH_2$, $(CH_3)_2NH$, $(C_3H_7)_2NH$, $(C_4H_9)_2NH$, $(C_5H_{11})_2NH$, $(C_6H_{13})_2NH$, $(CH_3)_3N$, $(C_2H_5)_3N$, $(C_3H_7)_3N$, $(C_4H_9)_3N$, $(C_5H_{11})_3N$, $(C_6H_{13})_3N$, $C_6H_5CH_2NH_2$, $HOCH_2CH_2NH_2$, $(HOCH_2CH_2)_2NH$, $(HOCH_2CH_2)_3N$, $C_2H_5NH(CH_2CH_2OH)$, $C_2H_5N(CH_2CH_2OH)_2$, $(HOH_2C)_3CNH_2$ and 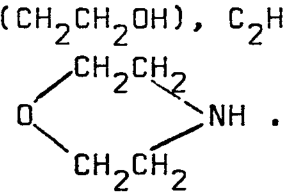 .

Further examples of other useful salts which can be prepared by the ion exchange technique are quaternary ammonium salts of the active substances i.e. salts in which the hydrogens in the active substances (structural formula I) have been substituted with quaternary ammonium ions such as $(CH_3)_4N$, $(C_2H_5)_4N$, $(C_3H_7)_4N$, $(C_4H_9)_4N$, $(C_5H_{11})_4N$, $(C_6H_{13})_4N$ and $C_2H_5N(CH_2CH_2OH)_3$. Lipophilic salts of this type can also be prepared by mixing a salt of the active substance with a quaternary ammonium salt in water and extracting out the resulting quaternary ammonium salt of the active substances with an organic solvent such as dichloromethane, chloroform, ethyl acetate, and methyl isobutyl ketone.

The compounds utilized within the invention may be formulated for use in human and veterinary medicine for therapeutic and prophylactic use. The compounds may be used in the form of a physiologically acceptable salt. Suitable salts are e.g. amine salts, e.g. dimethylamine and triethylamine salt, ammonium salt, tetrabutylammonium salt, cyclohexylamine salt, dicyclohexylamine salt; and metal salts, e.g. mono- and disodium salt, mono- and dipotassium salt, magnesium salt, calcium salt and zinc salt.

The compounds utilized within the invention are particularly useful for systemic treatment of virus infections, by oral administration or by injection.

In comparison with phosphonoformic acid, they are generally more stable in acid solutions, and are thus less readily decomposed in the stomach.

In clinical practice the compounds will normally be administered topically, orally, intranasally, by injection or by inhalation in the form of a pharmaceutical preparation comprising the active ingredient in the form of the original compound or optionally in the form of a pharmaceutically acceptable salt thereof, in association with a pharmaceutically acceptable carrier which may be a solid, semisolid or liquid diluent or an ingestible capsule, and such preparations comprise a further aspect of the invention. The compound may also be used without carrier material. As examples of pharmaceutical preparations may be mentioned tablets, drops such as nasal and eye drops, preparations for topical application such as ointments, jellies, creams and suspensions, aerosols for inhalation, nasal spray, liposomes etc. Usually the active substance will comprise between 0.05 and 99, or between 0.1 and 99 % by weight of the preparation, for example between 0.5 and 20 % for preparations intended for injection and between 0.1 and 50 % for preparations intended for oral administration.

To produce pharmaceutical preparations in the form of dosage units for oral application containing a compound of the invention the active ingredient may be mixed with a solid, pulverulent carrier, for example lactose, saccharose, sorbitol, mannitol, a starch such as potato starch, corn starch, amylopectin, laminaria powder or citrus pulp powder, a cellulose derivative or gelatine and also may include lubricants such as magnesium or calcium stearate or a Carbowax® or other polyethylene glycol waxes and compressed to form tablets or cores for dragées. If dragées are required, the cores may be coated for example with concentrated sugar solutions which may contain gum arabic, talc and/or titanium dioxide, or alternatively with a film forming agent dissolved in easily volatile organic solvents or mixtures of organic solvents. Dyestuffs can be added to these coatings, for example, to distinguish between different contents of active substance. For the preparation of soft gelatine capsules consisting of gelatine and, for example, glycerol as a plasticizer, or similar closed capsules, the active substance may be admixed with a Carbowax® or a suitable oil as e.g. sesam oil, olive oil, or arachis oil. Hard gelatine capsules may contain granulates of the active substance with solid, pulverulent carriers such as lactose, saccharose, sorbitol, mannitol, starches (for example potato starch, corn starch or amylopectin), cellulose derivatives or gelatine, and may also include magnesium stearate or stearic acid as lubricants.

By using several layers of the active drug, separated by slowly dissolving coatings sustained release tablets are obtained. Another way of preparing sustained release tablets is to divide the dose of the active drug into granules with coatings of different thickness and compress the granules into tablets together with the carrier substance. The active substance can also be incorporated in slowly dissolving tablets made for instance of fat and wax substances or evenly distributed in a tablet of an insoluble substance

such as a physiologically inert plastic substance.

In order to obtain dosage units of oral preparations - tablets, capsules, etc. - which are designed so as to prevent release of and possible decomposition of the active substance in the gastric juice, the tablets, dragées etc. may be enteric-coated, that is provided with a layer of a gastric juice-resistant enteric film or coating having such properties that it is not dissolved at the acidic pH in the gastric juice. Thus, the active substance will not be released until the preparation reaches the intestines. As examples of such known enteric coatings may be mentioned cellulose acetate phtalate, hydroxypropylmethylcellulose phtalates such as those sold under the trade names HP 55 and HP 50, and Eudragit®L and Eudragit®S.

Effervescent powders are prepared by mixing the active ingredient with non-toxic carbonates or hydrogen carbonates of e.g. sodium, potassium or calcium, such as calcium carbonate, potassium carbonate and potassium hydrogen carbonate, solid, non-toxic acids such as tartaric acid, ascorbic acid, and citric acid, and for example aroma.

Liquid preparations for oral application may be in the form of elixirs, syrups or suspensions, for example solutions containing from about 0.1 % to 20 % by weight of active substance, sugar and a mixture of ethanol, water, glycerol, propylene glycol and optionally aroma, saccharine and/or carboxymethylcellulose as a dispersing agent.

For parenteral application by injection preparations may comprise an aqueous solution of a water soluble pharmaceutically acceptable salt of the active acids according to the invention, desirably in a concentration of 0.5-10 %, and optionally also a stabilizing agent and/or buffer substances in aqueous solution. Dosage units of the solution may

advantageously be enclosed in ampoules.

For topical application, especially for the treatment of herpes virus infections on skin, genitals and in mouth and eyes the preparations are suitably in the form of a solution, ointment, gel, suspension, cream or the like. The amount of active substance may vary, for example between 0.05-20 % by weight of the preparation. Such preparations for topical application may be prepared in known manner by mixing the active substance with known carrier materials such as isopropanol, glycerol, paraffine, stearyl alcohol, polyethylene glycol, etc. The pharmaceutically acceptable carrier may also include a known chemical absorption promoter. Examples of absorption promoters are e.g. dimethylacetamide (U S Pat. No. 3,472,931), trichloroethanol or trifluroethanol (U S Pat. No. 3,891,757), certain alcohols and mixtures thereof (British Pat. No. 1,001,949). A carrier material for topical application to unbroken skin is also described in the British patent specification No. 1,464,975, which discloses a carrier material consisting of a solvent comprising 40-70 % (v/v) isopropanol and 0-60 % (v/v) glycerol, the balance, if any, being an inert constituent or a diluent not exceeding 40 % of the total volume of solvent.

The dosage at which the active ingredients are administered may vary within a wide range and will depend on various factors such as for example the severity of the infection, the age of the patient, etc. and may have to be individually adjusted. As a possible range for the amount of the active substance which may be administered per day may be mentioned from about 0.1 mg to about 2000 mg or from about 1 mg to about 2000 mg, or preferably from 1 mg to about 2000 mg for topical administration, from 50 mg to about 2000 mg or from 100 to about 1000 mg for oral administration and from 10 mg to about 2000 mg or from 50 to about 500 mg for injection. In severe cases it may be necessary to increase these doses

5-fold to 10-fold. In less severe cases it may be sufficient to use up to 500 or 1000 mg.

The pharmaceutical composistions containing the active ingredients may suitably be formulated so that they provide doses within these ranges either as single dosage units or as multiple dosage units and are preferably provided as sterile preparations.

Thus, it has been found according to the invention that the above compounds, and the physiologically acceptable salts thereof can be used to selectively inhibit the multiplication of viruses and the compounds and physiologically acceptable salts thereof are therefore useful in therapeutic and/or prophylactic treatment of virus infections and neoplastic diseases, as described above.

## Preparation of the active substances

References to "meaning given above" for $R_1$, $R_2$, $R_4$ and $R_5$ as used below refers to the definitions given in formula I. Hydroxycarbamoylphosphonic acid diesters are prepared by known methods. Examples of those methods are as described by T. Reetz *et al* in J. Am. Chem. Soc. 77 (1955) 3813 and in Houben-Weyl, Methoden der Organische Chemie, Auflage 4, Band XII/1, Organische Phosphorverbindungen, p. 453-458. Examples of such methods are the following.

A. Reacting a hydroxycarbonylphosphonic acid triester with ammonia or an amine, according to the formula:

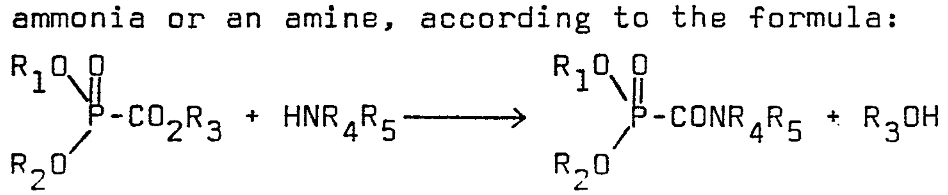

$R_1$, $R_2$, $R_4$ and $R_5$ have the meaning given above. $R_1$ and $R_2$ may be the same or different, and $R_4$ and $R_5$ may be the same

or different. $R_3$ is an aliphatic, cycloaliphatic, araliphatic or aromatic leaving group, such as alkyl groups containing 1-8 carbon atoms, cycloalkyl groups containing 3-8 carbon atoms, cycloalkyl-alkyl groups containing 4-8 carbon atoms, phenyl, and benzyl.

Preferentially the reaction is performed with ammonia, a primary amine or a suitable secondary amine, such as for example diethylamine, at a temperature from 0 to 100$^{\circ}$C for 1 hour to 5 days.

B. Reacting a phosphite triester with carbamoyl compounds according to the formula:

$$(R_1O)_3P + R_7\text{-}CONR_4R_5 \longrightarrow (R_1O)_2\overset{O}{\overset{\|}{P}}\text{-}CONR_4R_5 + R_1\text{-}R_7$$

$R_7$ is a leaving group suitable for Arbuzow type reactions, such as Cl, Br, I, sulphonate, carboxylate, alkoxide, and $R_1$, $R_4$, and $R_5$ have the meaning given above.

Preferentially the reaction is performed at 0 to 150$^{\circ}$C for 1 to 50 hours.

C. Reacting a phosphite triester with carbamoyl compounds according to the formula:

$$(R_1O)_2POR_8 + R_7\text{-}CONR_4R_5 \longrightarrow (R_1O)_2\overset{O}{\overset{\|}{P}}\text{-}CONR_4R_5 + R_8\text{-}R_7$$

wherein $R_1$, $R_4$, $R_5$ and $R_7$ have the meaning given above. $R_8$ may for example be an alkyl, a cycloalkyl, a cycloalkyl-alkyl, a benzyl, an adamantyl or any phosphite esterifying group suitable for participating in Arbuzow type reactions.

Preferentially the reaction is performed at 0 to 150$^{\circ}$C for 1 to 50 hours.

D. Reacting a phosphite triester with carbamoyl compounds according to the formula:

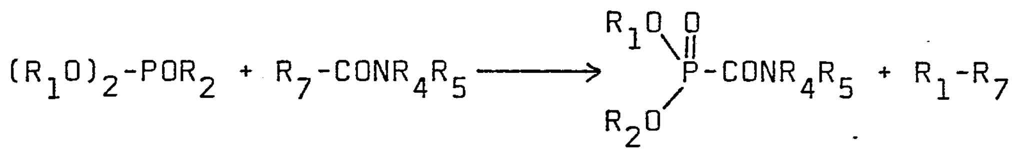

wherein $R_1$, $R_2$, $R_4$, $R_5$ and $R_7$ have the meaning given above, except that $R_1$ must not be phenyl.

Preferentially the reaction is performed at 0 to 150°C for 1 hour to 50 hours.

E. Reacting a phosphite diester salt with carbamoyl compounds according to the formula:

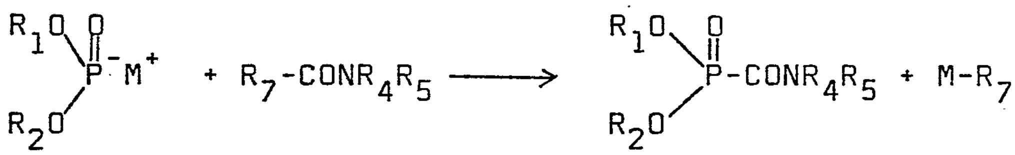

$R_1$, $R_2$, $R_4$, $R_5$ and $R_7$ have the meaning given above, and $R_1$ and $R_2$ may be the same or different. M is a cation, preferentially a metal such as Li, Na or K and the reaction is preferentially performed at 0 to 100°C for 1 to 50 hours in a solvent such as for example toluene, ether or tetrahydrofuran.

The phosphite diester salts are prepared by treating the phosphite diester with a suitable proton abstracting compound such as a metal alkoxide, suitably free from alcohol, such as lithium-, sodium- or potassium methoxide, ethoxide or t-butoxide or with a hydride such as sodium hydride or potassium hydride, or with a base such as butyllithium.

F. Reacting phosphite diesters with isocyanates according to the formula:

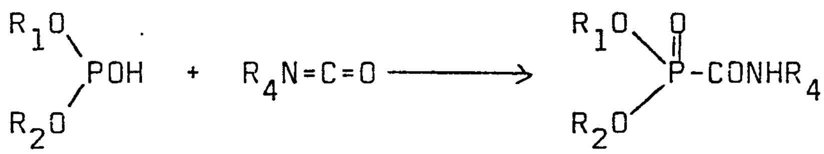

$R_1$, $R_2$ and $R_4$ have the meaning given above and $R_1$ and $R_2$ may be the same or different.

Preferentially the reaction is performed at 50 to 150$^oC$ for 1 to 50 hours.

The starting materials used in the above methods of preparation A-F are known compounds or may be prepared by known methods commonly used for the synthesis of hydroxycarbonylphosphonic acid triesters, phosphite esters, carbamoyl compounds and isocyanates. Examples of methods for the synthesis of phosphite esters may be found in Houben-Weyl, Methoden der Organischen Chemie, Auflage 4, Band XII/2, Organische Phosphorverbindungen, p. 5-78. Examples of methods for the synthesis of hydroxycarbonylphosphonic acid triesters are found in Houben-Weyl, Methoden der Organischen Chemie, Auflage 4, Band XII/1, Organische Phosphorverbindungen p.433-463.

G. Esterification of carbamoylphosphonic acid according to the formula:

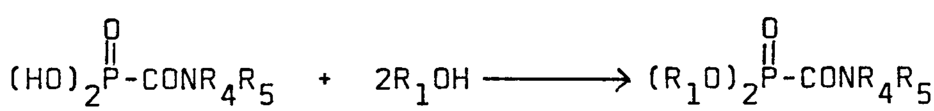

$R_1$, $R_4$ and $R_5$ have the meaning given above. The reaction is performed through the intermediary of activating agents known *per se* for the phosphorylation of alcohols and phenols. Examples of such methods are described for example by L. A. Slotin in Synthesis 1977, 737 and by H. Seliger and

H. Kössel in Progress in the Chemistry of Organic Natural Products 32 (1975) 297.

Synthesis of carbamoylphosphonic acids are described below in methods L-Q.

H. Esterification of carbamoylphosphonic acid monoester according to the formula:

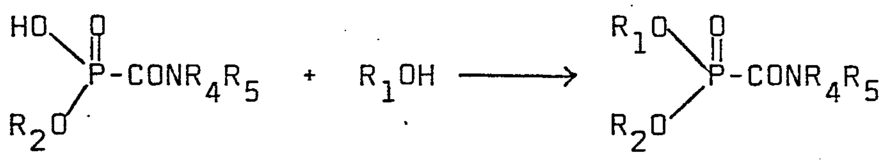

$R_1$, $R_2$, $R_4$ and $R_5$ have the meaning given above.

The reaction is performed through the intermediary of activating agents known per se for the phosphorylation of alcohols and phenols. Examples of such methods are described for example by L. A. Slotin in Synthesis 1977 737, and by H. Seliger and H. Kössel in Progress in the Chemistry of Organic Natural Products 32 (1975) 297. Synthesis of carbamoylphosphonic acid monoesters are described below in methods R-U.

J. Reacting carbamoylphosphonic acid dihalides according to the formula:

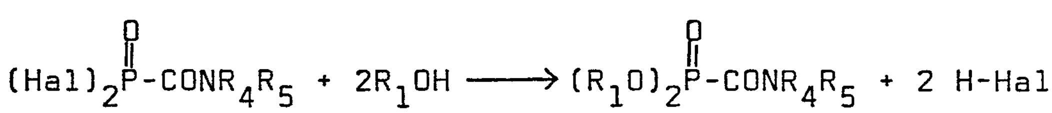

Hal is Cl, Br or I and $R_1$, $R_4$ and $R_5$ have the meaning given above.

The reaction is performed by methods known per se for the phosphorylation of alcohols and phenols by phosphoric and phosphonic acid halides. Examples of such methods are described for example by L. A. Slotin in Synthesis 1977 737,

and by H. Seliger and H. Kössel in Progress in the Chemistry of Organic Natural Products 32 (1975) 297.

Carbamoylphosphonic acid dihalides are prepared from carbamoylphosphonic acids by methods known per se for the synthesis of dihalides of phosphonic acids and phosphoric acids. References for those methods are found for example in the two publications above and in Houben-Weyl, Methoden der Organischen Chemie, Auflage 4, Band XII/1, p. 386-406 and Band XII/2, p. 211-225 and p. 274-292.

Carbamoylphosphonic acids are prepared by methods described below in L-Q.

K. Reacting carbamoylphosphonic acid monoester halide according to the formula:

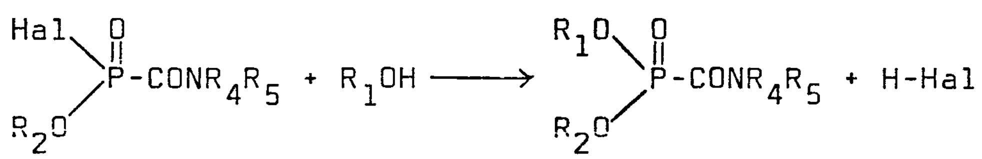

Hal is Cl, Br or I and $R_1$, $R_2$, $R_4$ and $R_5$ have the meaning given above.

The reaction is performed by methods known per se for the phosphorylation of alcohols and phenols. Examples of such methods are described for example by L. A. Slotin in Synthesis 1977, 737 and by H. Seliger and H. Kössel in Progress in the Chemistry of Organic Natural Products 32 (1975) 297.

Carbamoylphosphonic acid monoester halides are prepared from carbamoylphosphonic acid monoesters by methods known per se for the synthesis of monohalides of phosphonic and phosphoric acids. References for those methods are found for example in the two publications above and in Houben-Weyl,

Methoden der Organischen Chemie, Auflage 4, Band XII/1, p. 386-406 and Band XII/2 p. 211-225 and p. 274-292.

Carbamoylphosphonic acid monoesters are prepared by methods described below in R-U.

Carbamoylphosphonic acids may be prepared by known methods such as:

L. Hydrolysis of carbamoylphosphonic acid diesters according to the formula:

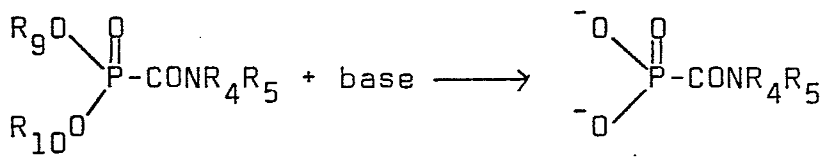


$R_4$ and $R_5$ have the meaning given above. $R_9$ and $R_{10}$ have the meaning given $R_1$ and $R_2$. $R_9$ and $R_{10}$ may be the same or different.

Preferably the reaction is performed with a base such as $MHCO_3$, $M_2CO_3$ or MOH. $M^+$ is for example $NH_4^+$ or a metal such as $Li^+$, $Na^+$ or $K^+$. Preferably the reaction is performed as an aqueous hydrolysis at 20 to 100$^oC$ for 1 to 24 hours.

The carbamoylphosphonic acid diesters may be prepared by methods analogous to those described above in A-K.

Optionally the disalt of the phosphonate group may be acidified to phosphonic acid groups by acidification with an acid, such as a mineral acid or a strong cation exchanger ($H^+$).

M. Aqueous hydrolysis of carbamoylphosphonic acid diesters according to the formula

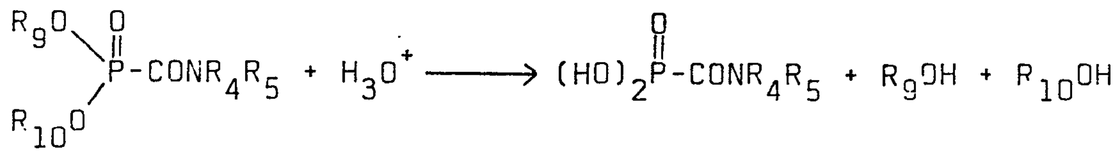

$R_4$, $R_5$, $R_9$ and $R_{10}$ have the meaning given above.

Preferably the reaction is performed with an acid such as HCl or $H_2SO_4$.

Optionally the phosphonate acid groups may be neutralized. Preferably they may be neutralized with a weak cation exchanger ($M^+$) or with a base such as $MHCO_3$, $M_2CO_3$ or MOH. $M^+$ is $NH_4^+$ or a metal such as $Li^+$, $Na^+$ or $K^+$.

N. Reacting carbamoylphosphonic acid diesters with hydrohalide acids according to the formula:

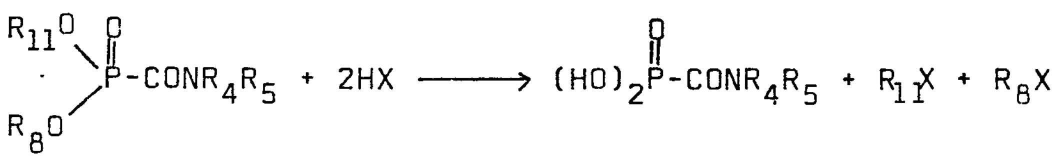

$R_4$, $R_5$ and $R_8$ have the meaning given above. $R_{11}$ has the meaning given $R_8$ and $R_8$ and $R_{11}$ may be the same or different. X is Cl, Br or I.

Preferably HI may be used and the reaction may preferably be performed in a dry solvent such as acetic acid or methylene chloride at a temperature from 0 to 30°C.

Optionally the phosphonic acid groups may be neutralized. Preferably a weak cation exchanger ($M^+$) or a base such as $MHCO_3$, $M_2CO_3$ or MOH is used. $M^+$ is for example $NH_4^+$ or a metal such as $Li^+$, $Na^+$ or $K^+$.

The carbamoylphosphonic acid diesters are prepared by methods analogous to those described above in A-K.

O. Aqueous hydrolysis of a carbamoylphosphonic acid containing two silyl esterified phosphonate groups, according to the formula:

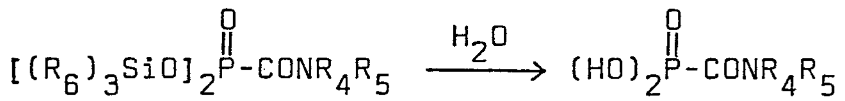

$R_4$ and $R_5$ have the meaning given above, and $R_6$ is an inert organic residue, preferably an organic group such as for example $CH_3$. Other examples of silyl ester groups which may be used are for example butyldiphenylsilyl compounds, which have been described by R. A. Jones _et_ _al_ in Biochemistry _17_ (1978) 1268 as phosphate ester derivatives.

Optionally the formed phosphonic acid groups can be neutralized. Preferably they may be neutralized with a weak cation exchanger ($M^+$) or with a base such as $MHCO_3$, $M_2CO_3$ or MOH. $M^+$ is $NH_4^+$ or a metal such as $Li^+$, $Na^+$ or $K^+$.

The phosphonate bis-silyl esters may be obtained according to the formula

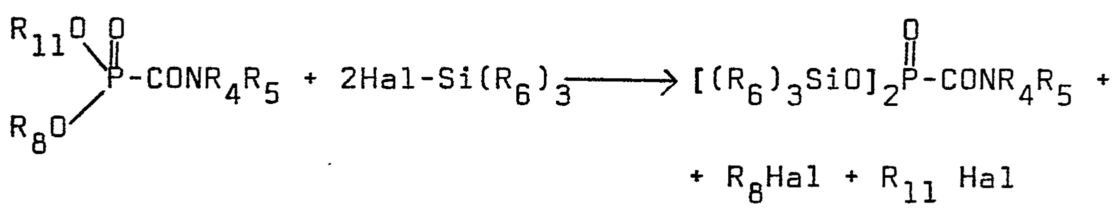

$R_4$, $R_5$, $R_6$, $R_8$ and $R_{11}$ have the meaning given above.

Preferably the organic residues of the silyl group are as described above. Hal is Cl, Br or I and preferably the reaction is performed at -20°C to reflux temperatures for 1 hour to several days.

The carbamoylphosphonic acid diesters are prepared by methods analogous to those described above in A-K.

Alternatively the bissilylphosphonate esters may be prepared by reacting a trissilylphosphite with an activated carbamoyl compound according to the formula:

$$[(R_6)_3SiO]_3P + R_7\text{-}CONR_4R_5 \longrightarrow [(R_6)_3SiO]_2\overset{O}{\overset{\|}{P}}\text{-}CONR_4R_5 + (R_6)_3Si\text{-}R_7$$

$R_4$, $R_5$, $R_6$ and $R_7$ have the meaning given above and preferentially the organic residues of the silyl group are as described above. Preferentially the reaction is performed at 20-150°C for 1 to 25 hours.

The tris-silylphosphites are prepared by known methods, as described for example by Herrin et al in J. Med. Chem. 20 (1977) 660, for the preparation of tris(trimethylsilyl)-phosphite.

P. Hydrogenating dibenzyl carbamoylphosphonates according to the formula:

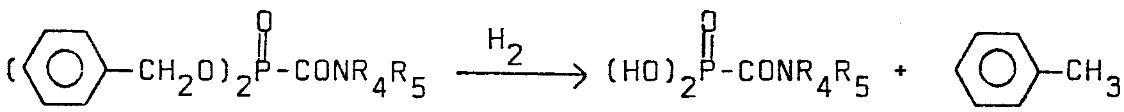


$R_4$ and $R_5$ have the meaning given above.

Preferably the reaction may be performed with a catalyst such as palladium-carbon. Optionally the phosphonic acid groups may be neutralized. Preferably they may be neutralized with a weak cation exchanger ($M^+$) or with a base such as $MHCO_3$, $M_2CO_3$ or MOH. $M^+$ is for example $NH_4^+$ or a metal such as $Li^+$, $Na^+$ or $K^+$.

Q. Reacting carbamoylphosphonic acid monoesters to carbamoylphosphonic acids by procedures analogous to those described above in L-P.

Optionally the carbamoylphosphonic acids thus obtained may be neutralized with a weak cation exchanger ($M^+$) or with a base such as $MHCO_3$, $M_2CO_3$ or MOH. $M^+$ is for example $NH_4^+$ or a metal such as $Li^+$, $Na^+$ or $K^+$.

The carbamoylphosphonic acid monoesters may be prepared by methods described below in R-U.

Carbamoylphosphonic acid monoesters are prepared by known methods, such as

R. Reacting a carbamoylphosphonic acid diester with an iodide or bromide anion according to the formula

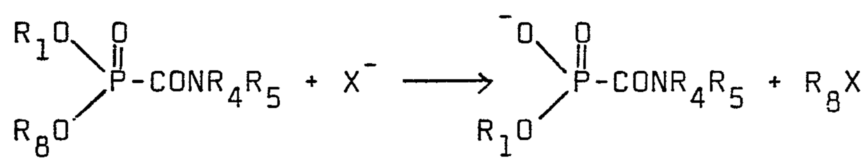

$R_1$, $R_4$, $R_5$ and $R_8$ have the meaning given above. $X^-$ is $Br^-$ or $I^-$.

Preferably the reaction is carried out with sodium iodide in a solvent such as for example tetrahydrofuran or acetone. Preferably the reaction is carried out at a temperature from 20 to 100°C from 2 hours to 7 days.

The carbamoylphosphonic acid diesters may be prepared by methods analogous to those described above in A-K.

S. Hydrolyzing the carbamoylphosphonic acid diester with a base according to the formula

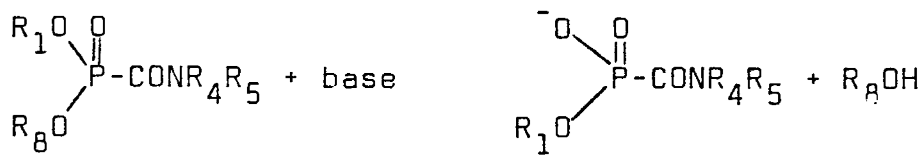


$R_1$, $R_4$, $R_5$ and $R_8$ have the meaning given above.

Preferably the reaction is carried out with a base such as for example sodium hydrogencarbonate, sodiumcarbonate or sodium hydroxide in water at a temperature from 20 to 100$^oC$ from 2 hours to 7 days.

The carbamoylphosphonic acid diesters may be prepared by methods analogous to those described in A-K.

T. Aqueous hydrolysis of a carbamoylphosphonic acid diester, containing a silylesterified phosphonate group, according to the formula:

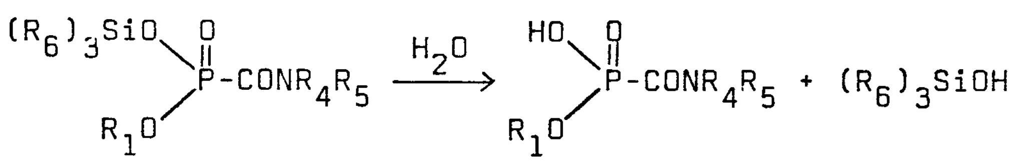


wherein $R_1$, $R_4$ and $R_5$ have the meaning given above and $R_6$ is an inert organic residue and preferentially is as described in method O.

Optionally the formed phosphonic acid group may be neutralized. Preferably it may be neutralized with a base such as for example $MHCO_3$, $M_2CO_3$ or MOH or with a weak cation exchanger ($M^+$), where $M^+$ is $NH_4^+$ or a metal such as $Li^+$, $Na^+$ or $K^+$.

The silyl esterified phosphonate group may be obtained by treating the carbamoylphosphonic acid diester with a halosilane according to the formula:

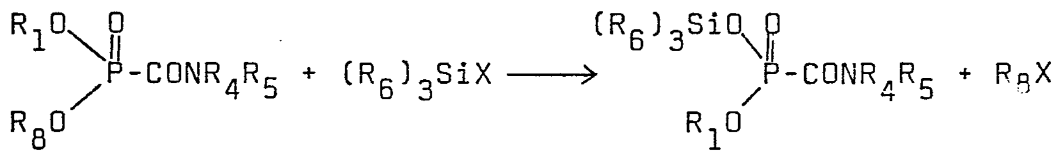

X is Cl, Br or I and $R_1$, $R_4$, $R_5$, $R_6$ and $R_8$ have the meaning given above.

Preferably the reagents used for silylation are for example bromotrimethylsilane at -20$^{o}$C to 50$^{o}$C for 1/2 to 20 hours, or alternatively for example chlorotrimethylsilane at 20$^{o}$C to reflux temperature for several days.

The carbamoylphosphonic acid diesters are prepared by methods analogous to those described above in A-K.

Alternatively the silyl esterified phosphonate group may be prepared by reacting a phosphite triester containing two silyl ester groups, with a carbamoylchloride, according to the formula:

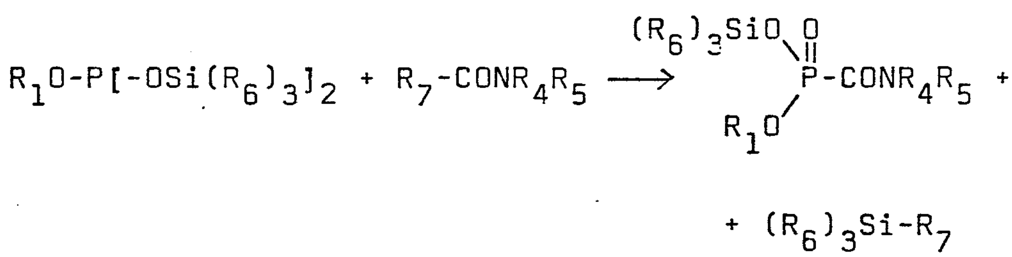

wherein $R_1$, $R_4$, $R_5$, $R_6$ and $R_7$ have the meaning given above.

Preferably the phosphite is an ester such as for example a bis-(trimethylsilylated)phosphite triester. These compounds can be prepared by methods known per se. For example the synthesis of propyl- and hexyl-bis(trimethylsilyl)phosphites are described in T. R. Herrin et al, J. Med. Chem. 20 (1977) 660.

U. Reacting carbamoylphosphonic acid according to the formula:

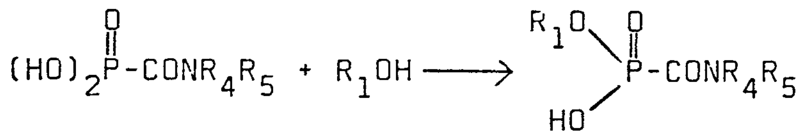

$R_1$, $R_4$ and $R_5$ have the meaning given above. The reaction is performed through the intermediary of activating agents known per se for the phosphorylation of alcohols and phenols. Examples of such methods are described for example by L.A. Slotin in Synthesis 1977, 737 and by H. Seliger and H. Kössel in Progress in the Chemistry of Organic Natural Products, 32 (1975) 297.

Synthesis of carbamoylphosphonic acids are described above in methods L-Q.

## Examples

Preparation of carbamoylphosphonic acid diesters.

### Example 1. Diethyl carbamoylphosphonate

Ammonia gas was passed into 19.6 g of diethyl methoxycarbonylphosphonate at 40°C for 3 hours. After a while crystals appeared. The reaction mixture was left over night. The precipitate was filtered and crystallized twice from benzene and then air dried. Yield 10.8 g, m.p. 132-3°C. Synthesized according to T. Reetz et al, J. Am. Chem. Soc. 77 (1955) 3813.

Example 2

Analogously the following compounds were prepared, by adding the appropriate amine to the hydroxycarbonylphosphonic acid triester at 0-50°C followed by heating at 20-50°C for 1 to 50 hours.

a) Diethyl ethylcarbamoylphosphonate

Synthesized according to T. Reetz *et al*, J. Am. Chem. Soc. 77 (1955) 3813, from diethyl methoxycarbonylphosphonate and ethylamine. B.p.$_{0.6-0.8}$ 117-120°C.

NMR ($CDCl_3$): 1.0-1.4 ($CH_3$), 3.1-3.55 (N-$CH_2$), 3.95-4.45 ($OCH_2$), 7.8 (NH).

IR (neat) $cm^{-1}$: 1660 (CO), 1280, 1230, 1060, 1030, 980.

b) Diethyl benzylcarbamoylphosphonate

From diethyl methoxycarbonylphosphonate and benzylamine (40-60°C, about 1 hour, room temp. 48 hours). B.p.$_{0.02}$ 140-170°C. Rechrystallized from isopropyl ether m.p. 53-5°C.

Analyses for $C_{12}H_{18}NO_4P$. Found (calculated): C 53.16 (53.13), H 6.70 (6.69), N 5.11 (5.16).

NMR ($CDCl_3$) $\delta$: 1.30 (t, J 7Hz, $CH_3$), 3.93-4.53 (C-$CH_2$ and Ar-$CH_2$), 7.35 (s, $C_6H_5$), 7.9 (NH).

IR (KBr) $cm^{-1}$: 1650, 1260, 1210, 1040, 1010, 960, 940.

## Example 3. Diethyl diethylcarbamoylphosphonate

Synthesized according to T. Reetz *et al*, J. Am. Chem. Soc. 77 (1955) 3813.

12.5 g of diethyl carbamoylchloride was added over 45 min. to 20 g of triethylphosphite at 130-135°C. The heating was continued for 3 hours and the product was distilled. Yield 18.7 g (86 %). B.p.$_{0.7}$ 99-102°C. IR (neat) $cm^{-1}$: 1620 (CO), 1280, 1250, 1050, 1020, 980.

## Example 4. Diphenyl benzylcarbamoylphosphonate

0.71 g of diphenyl ethyloxycarbonylphosphonate [A. Takamizawa and Y. Sato, Chem. Pharm. Bull. 12 (1964) 398] and 1.5 g of benzylamine were heated at 140°C overnight. Volatile components were evaporated *in vacuo* ($10^{-3}$ mm) at room temperature and the residue was extracted with about 40 ml of di-isopropylether. The solution was evaporated *in vacuo* and the residue was washed with 3x25 ml of hexane and dried (0.30 g). About 70 mg of this residue was purified by preparative layer chromatography (silica gel 20x20 cm, chloroform-ethanol, 24-1) to give diphenyl benzylcarbamoylphosphonate. T.l.c. (silica gel, chloroform--ethanol, 24-1) $R_f$ 0.30 (purity about 95 %). IR (neat) $cm^{-1}$: 1630, 1590, 1450, 1260, 1090, 1060, 1030.

Preparation of carbamoylphosphonic acids

Example 5. Ethylcarbamoylphosphonic acid

Diethyl ethylcarbamoylphosphonate (synthesized according to T. Reetz et al, J. Am. Chem. Soc. 77 (1955) 3813) (2.09 g) and NaOH (1.2 g) in 24 ml of $H_2O$ were heated at 100$^oC$ for 1 hour and left at room temperature for another 24 hours. The solution was evaporated in vacuo to an oily residue which did not crystallize from ethanol. It was redissolved in hot water (20 ml) and cooled on ice. The precipitate (0.8 g) was discarded by filtration and the water solution was passed over a column with Dowex 50 $H^+$ (150 ml). The eluate was evaporated in vacuo to yield colourless crystals of the desired product. M.p. 165-7$^oC$.

Thin layer chromatography (polyethyleneimine, 1M LiCl, molybdate spray) $R_f$ 0.63, about 95 % purity.

A second batch of the synthesis gave a yield of 0.85 g of ethylcarbamoylphosphonic acid, m.p. 158-161$^oC$.
T.l.c. $R_f$ 0.63. By t.l.c. the compound was estimated to contain <0.2 % of trisodium oxycarbonylphosphonate.

NMR (DMSO-$d_6$)$\delta$: 1.03 (t, J 7Hz, $CH_3$), 3.15 (quintet, J 7Hz, $CH_2$), 8.4 (broad, NH), 10.7 (broad, 2OH).

Example 6.

Analogously, as described in example 5, benzylcarbamoylphosphonic acid was prepared and analyzed.

From diethyl benzylcarbamoylphosphonate.
After passage through a Dowex 50 ($H^+$) ion exchanger, the product was recrystallized from ethanol-isopropylether. M.p. (dec) 178-180$^oC$. Two more recrystallizations gave m.p. (dec) 180-2$^oC$.

Analysis for $C_8H_{10}NO_4P$. Found (calculated): C 44.66 (44.66), H 4.70 (4.68), N 6.49 (6.51). T.l.c. $R_f$ 0.43. By t.l.c. the compound was estimated to contain $<$0.2 % of trisodium oxycarbonylphosphonate.

NMR (DMSO-$d_6$)$\delta$: 4.30 (d, J 6Hz, $CH_2$), 7.27 (s, $C_6H_5$), 9.00 (t, J 6Hz, NH), 9.95 (s, 2OH).

Example 7. Disodium diethylcarbamoylphosphonate

2.37 g (10 mmole) of diethyl diethylcarbamoylphosphonate and 1.2 g (30 mmole) of sodium hydroxide in 25 ml of water was heated at reflux for 2 hours. The solution was passed over a column of Dowex 50 ($H^+$), eluted with about 75 ml of water, and the eluate was treated with about 60 g of Amberlite IRC 50 ($Na^+$). The solution was evaporated *in vacuo* to give 0.93 g of disodium diethylcarbamoylphosphonate. T.l.c. (polyethyleneimine , 1M LiCl, molybdate spray) $R_f$ 0.68. By t.l.c. a second spot could be detected which could be trisodium oxycarbonylphosphonate (about 5 %). IR (KBr) $cm^{-1}$: 1590, 1440, 1150, 1130, 1110, 1000.

Preparation of carbamoylphosphonic acid monoesters.

Example 8. Ethyl sodium benzylcarbamoylphosphonate

0.71 g (2.6 mmole) of diethyl benzylcarbamoylphosphonate and 5.0 ml of 0.5N NaOH (2.5 mmole) were stirred at room temperature for 24 hours. The solution was washed with chloroform and evaporated *in vacuo*. The residue was treated with chloroform to give 0.58 g (88 %) of ethyl sodium benzylcarbamoylphosphonate as an amorphous residue. T.l.c. (silica gel, ethanol, UV and $J_2$) $R_f$ 0.49, single spot. NMR ($D_2O$)$\delta$: 1.23 (t, J 7Hz, $CH_3$), 4.00 (quintet, J 7Hz, $CH_2$), 4.48 (s, N-$CH_2$), 7.38 (s, $C_6H_5$). IR(KBr)$cm^{-1}$: 1630, 1240, 1100, 1060, 960.

Pharmaceutical compositions

The following examples illustrate the preparation of pharmaceutical compositions of the invention. The active substance is preferably used in the form of its sodium salt, when salt formation is possible.

Example 9. Aerosol for inhalation

| | | |
|---|---|---|
| Active substance | | 1.00 g |
| Miglyol® | | 0.20 g |
| Frigen® 11/12/113/114 | ad | 100.0 g |

Example 10. Tablets

Each tablet contains:

| | |
|---|---|
| Active substance | 20.0 mg |
| Maize starch | 25.0 mg |
| Lactose | 190.0 mg |
| Gelatin | 1.5 mg |
| Talc | 12.0 mg |
| Magnesium stearate | 1.5 mg |
| | 250.0 mg |

Example 11. Suppositories

Each suppository contains:

| | |
|---|---|
| Active substance | 20.0 mg |
| Ascorbyl palmitate | 1.0 mg |
| Suppository base (Imhausen H or Witespol® H) ad | 2000.0 mg |

0003008

Example 12. Syrup

| | |
|---|---|
| Active substance | 0.200 g |
| Liquid glucose | 30.0 g |
| Sucrose | 50.0 g |
| Ascorbic acid | 0.1 g |
| Sodium pyrosulfite | 0.01 g |
| Disodium edetate | 0.01 g |
| Orange essence | 0.025 g |
| Certified colour | 0.015 g |
| Purified water | ad 100.0 g |

Example 13. Injection solution

| | |
|---|---|
| Active substance (as its sodium salt) | 0.500 mg |
| Sodium pyrosulfite | 0.500 mg |
| Disodium edetate | 0.100 mg |
| Sodium chloride | 8.500 mg |
| Sterile water for injection | ad 1.00 ml |

Example 14. Solution for inhalation

| | |
|---|---|
| Active substance (as its sodium salt) | 5.00 g |
| Disodium edetate | 0.10 g |
| Sodium chloride | 0.85 g |
| Hydrochloric acid to pH 6.5-6.9 | |
| Purified water | ad 100.0 ml |

Example 15. Drops

| | |
|---|---|
| Active substance (as its sodium salt) | 2.00 g |
| Citric acid | 1.00 g |
| Disodium edetate | 0.10 g |
| Liquid glucose | 50.00 g |
| Ethanol 95 % | 10.00 g |
| Sodium hydroxide and hydrochloric acid to pH 6.2-6.8 | |
| Purified water | ad 100.0 ml |

Example 16. Solution for topical use

| | |
|---|---|
| Active substance (as its sodium salt) | 2.00 g |
| Isopropanol | 38.0 g |
| Glycerol | 13.6 g |
| Hydrochloric acid to pH 5.0-7.0 | |
| Purified water | ad 100.0 g |

Preparations containing 0.2, 0.5 and 1.0 g of active substance have also been prepared.

Example 17. Jelly

| | |
|---|---|
| Active substance (as its sodium salt) | 4.0 g |
| Methocel® | 4.0 g |
| Methyl paraoxybenzoate | 0.12 g |
| Propyl paraoxybenzoate | 0.05 g |
| Sodium hydroxide and hydrochloric acid to pH 6.7 | |
| Distilled water | ad 100.0 ml |

Example 18. Ointment I

| | |
|---|---|
| Active substance (as its sodium salt) | 2.5 g |
| Cetyltrimethylammonium bromide | 0.6 g |
| Stearyl alcohol | 2.25 g |
| Cetanol | 6.75 g |
| Liquid paraffin | 17.0 g |
| Glycerol | 12.0 g |
| Hydrochloric acid to pH 6.5 | |
| Distilled water | ad 100.0 g |

Preparations containing 0.2, 0.5, 1.0 and 2.0 g of active substance have also been prepared.

Example 19. Inhalation solution

| | | |
|---|---|---|
| Active substance (as its sodium salt) | | 5.00 g |
| Sodium pyrosulfite | | 0.10 g |
| Disodium edetate | | 0.10 g |
| Sodium chloride | | 0.85 g |
| Purified water | ad | 100.0 ml |

Example 20. Sublingual tablets

| | |
|---|---|
| Active substance (as its sodium salt) | 5.0 mg |
| Lactose | 85.0 mg |
| Talc | 5.0 mg |
| Agar | 5.0 mg |
| | 100.0 mg |

Example 21. Drops

| | | |
|---|---|---|
| Active substance (as its sodium salt) | | 2.00 g |
| Ascorbic acid | | 1.00 g |
| Sodium pyrosulfite | | 0.10 g |
| Disodium edetate | | 0.10 g |
| Liquid glucose | | 50.00 g |
| Absolute alcohol | | 10.00 g |
| Purified water | ad | 100.0 ml |

Example 22. Syrup

| | | |
|---|---|---|
| Active substance (as its sodium salt) | | 0.200 g |
| Liquid glucose | | 30.0 g |
| Sucrose | | 50.0 g |
| Ascorbic acid | | 0.1 g |
| Disodium edetate | | 0.01 g |
| Orange essence with solubilizer | | 0.25 g |
| Hydrochloric acid to pH 6.0-6.5 | | |
| Purified water | ad | 100.0 g |

Example 23. Solution for injection

| | | |
|---|---|---|
| Active substance (as its sodium salt) | | 0.500 mg |
| Disodium edetate | | 0.100 mg |
| Sodium chloride | | 8.500 mg |
| Hydrochloric acid to pH 6.5-7.0 | | |
| Sterile water for injection | ad | 1.00 ml |

Example 24. Ointment II

| | | |
|---|---|---|
| Active substance (as its sodium salt) | | 2.5 g |
| Polyethylene glycol 1500 | | 50.0 g |
| Polyethylene glycol 4000 | | 15.0 g |
| Propylene glycol | ad | 100.0 g |

Example 25. Ointment III

| | | |
|---|---|---|
| Active substance (as its sodium salt) | | 3.0 g |
| Sorbitan monoleate | | 5.0 g |
| Petrolatum | ad | 100.0 g |

Example 26. Gastric juice-resistant tablets

Tablets according to examples 10 and 20 are coated with an enteric coating solution with the following composition:

| | | |
|---|---|---|
| Cellulose acetate phtalate | | 120.0 g |
| Propylene glycol | | 30.0 g |
| Sorbitan monoleate | | 10.0 g |
| Ethanol 95 % | | 450.0 ml |
| Acetone | q.s. ad | 1000.0 ml |

The coating is carried out by a pouring procedure in a conventional coating pan or by spraying the tablets in a pan spray tablet coater.

Example 27. Eye ointment

| | |
|---|---|
| Active substance | 5 g |
| Paraffine oil | 19 g |
| Petrolatum | 76 g |

Example 28. Cream

| | |
|---|---|
| Active substance | 3.0 g |
| Arlaton® | 4.0 g |
| Cetanol | 2.0 g |
| Stearic acid | 2.0 g |
| Paraffine oil | 2.0 g |
| Propylene glycol | 2.0 g |
| Glycerol | 1.5 g |
| Methyl-p-hydroxybensoate | 0.06 g |
| Propyl-p-hydroxybensoate | 0.03 g |
| Sodium hydroxide | 0.002 g |
| Hydrochloric acid 2 M to pH 8.0 (water phase) | |
| Distilled water | ad 100 g |

Example 29. Jelly

| | |
|---|---|
| Active substance | 3.0 g |
| Methocel® | 2.45 g |
| Glycerol | 10.0 g |
| Tween® | 0.10 g |
| Methyl-p-hydroxybensoate | 0.06 g |
| Propyl-p-hydroxybensoate | 0.03 g |
| Sodium hydroxide | 0.002 g |
| Hydrochloric acid 2 M to pH 8.0 | |
| Distilled water | ad 100 g |

## Biological tests

### I. Inhibition of virus multiplication in cell culture

#### A. Inhibition of herpes simplex type 1 plaque

The plaque reduction assay for herpes simplex type 1 was performed on GMK (Green Monkey Kidney) cells as described by Ejereito *et al*, J. Gen. Virol. 2 (1968) 357. Monolayers on 5 cm petri dishes were used and after virus adsorption the test compound was added in the medium. The results are shown below.

Inhibition of herpes simplex type 1 plaque on GMK monolayers

| Test compound 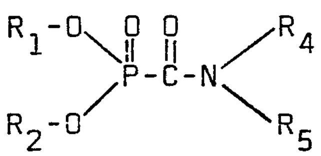 | | | | Concentration of test compound (µM) | Inhibition (%) |
|---|---|---|---|---|---|
| $R_1$ | $R_2$ | $R_4$ | $R_5$ | | |
| $C_2H_5$ | $C_2H_5$ | $C_2H_5$ | $C_2H_5$ | 500 | 0;83 |
| $C_2H_5$ | $C_2H_5$ | H | $-CH_2-$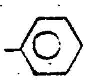 | 500 | 59;0 |

B. Inhibition of influenza (WSN Wilson Smith Neurotropic type A.) plaque

The method for plaque assay of influenza has been described by Bentley *et al*, Archiv für die Gesamte Virusforschung 33 (1971) 234.

Monolayers of MDCK (Madin Darby Canine Kidney) cells on 5 cm plastic petri dishes were inoculated with 100 plaque-forming units of influenza virus (WSN). After virus adsorption, 5 ml of agarose overlay containing different concentrations of the test compound were added and the plates were incubated at 34°C for 4 days. The plaques formed at this time were counted. The results are shown below.

Inhibition of influenza (WSN Wilson Smith Neurotropic type A) plaque on GMK monolayers.

| Test compound 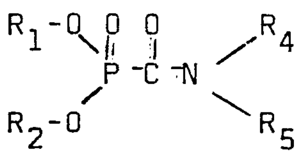 | | | | Code | Conc. of test compound (μM) | Inhibition (%) |
|---|---|---|---|---|---|---|
| $R_1$ | $R_2$ | $R_4$ | $R_5$ | | | |
| $C_2H_5$ | $C_2H_5$ | $C_2H_5$ | $C_2H_5$ | VIS 228 | 500 | 25; 37 |
| $C_2H_5$ | $C_2H_5$ | H | $-CH_2-$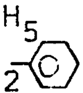 | VIS 229 | 500 | 51; 0 |

II. Stability test

The acid stability was investigated by dissolving 5 mg of each compound in 1 ml of 0.1 N HCl in a test tube. For use as references 0.2 ml of each solution was withdrawn, immediately treated with 0.2 ml of a 10 % aqueous solution of $NaHCO_3$ and frozen. The remaining 0.8 ml of each solution was incubated at 37°C for 2 hours. After incubation, 0.8 ml of a 10 % aqueous solution of $NaHCO_3$ was added to each solution and the solutions were frozen. The incubated compounds and the reference compounds were lyophilized to dryness and redissolved in distilled $H_2O$, 0.2 ml and 1.0 ml respectively, for each reference solution and incubated solution. The solutions were applied to silica gel (Merck $PF_{254}$, 20x20 cm) and polyethylene imine (Macherey-Nagel PEI, 20x20 cm) thin layer plates. A total of 20 µl of the reference solutions (100 µg compound) and 25 µl of the incubated solutions (100 µg compound) were applied. To each plate was also added, as references, solutions of phosphorous acid ($H_2HPO_3$) (5 and 20 µg) and of trisodiumphosphonoformate (5 and 20 µg). (Decomposition of phosphonoformic acid at low pH produces phosphorous acid).

The silica gel plates were prepared in duplicate and eluted with a solution composed of methanol - 10% aq ammonia - trichloroacetic acid - water (50-15-5-3, v/v) and the polyethylene imine plates were eluted with a 1M aq lithium chloride solution. After elution the plates were dried. One of the duplicated silica gel plates was sprayed with 4 % aq $(NH_4)_2MoO_4$ and the polyethylene imine plates were sprayed with a solution composed of 60 % $HClO_4$ - 0.1N aq HCl - 4% aq $(NH_4)_2MoO_4$ - $H_2O$ (5-10-25-60, v/v). The silica gel plates were briefly dried at 80-90°C and sprayed with 1% $SnCl_2$ in 10% aq HCl. Phosphorous acid and phosphonic acid groups appeared as blue spots on the silica gel plates (System I) and as white spots on the polyethylene imine plates (System II). The remaining duplicate silica gel plates were exposed to iodine vapor for detection of di- and triesters of phosphonoformic acid.

| | $R_f$ System I | $R_f$ System II |
|---|---|---|
| Phosphorous acid | 0.31 | 0.71 |
| $Na_3$-phosphonoformate | 0 | 0.21 |

The formation of phosphorous acid and phosphonoformic acid in each incubated solution was estimated and the results are given below. The figures for the non-incubated reference compounds are given in parenthesis.

| Test compound 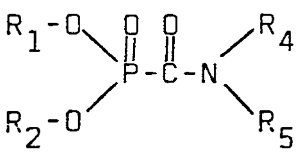 | | | | Estimated formation of phosphorous acid (µg) | Estimated formation of $Na_3$-phosphonoformate (µg) |
|---|---|---|---|---|---|
| $R_1$ | $R_2$ | $R_4$ | $R_5$ | | |
| Na | Na | H | H | 15 (5) | N.D. (N.D.) |
| $C_2H_5$ | $C_2H_5$ | $C_2H_5$ | $C_2H_5$ | N.D. (N.D.) | N.D. (N.D.) |
| $C_2H_5$ | $C_2H_5$ | H | H | N.D. (N.D.) | N.D. (N.D.) |
| $C_2H_5$ | $C_2H_5$ | H | $C_2H_5$ | N.D. (N.D.) | N.D. (N.D.) |
| $C_2H_5$ | $C_2H_5$ | H | $CH_2$- 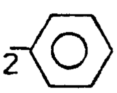 | N.D. (N.D.) | N.D. (N.D.) |
| Trisodiumphosphonoformate (reference) | | | | >20 (N.D.) | |

## III. In vivo metabolization

Metabolization of compounds of the invention was tested in NMR I 19-20 g male mice. The test compound (10 μmol) was dissolved in 0.5 ml saline and injected intraperitoneally. Two mice kept in one cage (for metabolization experiment) were used for each compound. The urine obtained from the two mice on day 1, 2 and 3 after the injections was collected. The urine was diluted with Tris-HCl buffer (pH 7.9) to a constant volume of 1 ml. This volume was then diluted 1:500, 1:5000 and 1:50000 with the same buffer and assayed for phosphonoformic acid activity on cell-free influenza polymerase. The assay mixture which includes $Mn^{2+}$ and assay conditions are described by Bishop, Obijeski and Simpson, J. Virol. 6, 66 (1971). Phosfonoformic acid in diluted urine gave 50% inhibition at 0.5 μM in this assay and was used as a standard to estimate the amount of phosphonoformic acid activity formed in the urine from compounds of the invention.

| Test compound | Code | Recovered phosphonoformic acid activity in urine (μmol phosphonoformic acid) | | |
|---|---|---|---|---|
| | | Day 1 | Day 2 | Day 3 |
| 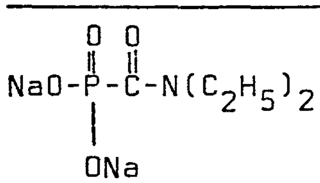 | VIS 232 | 0.63 | 0.36 | <0.01 |
| 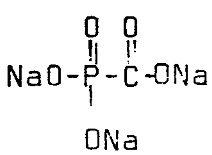 | (reference) | 1.25 | 0.13 | <0.01 |

Discussion of test results

As seen in test I compounds of the invention are active on herpes virus and influenza virus multiplication in cells. According to the stability test II, compounds of the invention are more stable than trisodium phosphonoformate in 0.1 M aqueous HCl, which is a model for stability in gastric juice, and the compounds of the invention should therefore be more suitable for oral administrations than phosphonoformic acid and physiologically acceptable salts thereof. The test on in vivo metabolism III shows that compounds of the invention are metabolized to phosphonoformic acid measured as phosphonoformic acid activity on influenza polymerase. It is also shown in test III that compounds according to the invention can give such an active metabolite in the urine of mice over a longer time period than trisodium phosphonoformate. Thus compounds of the invention have a prolonged activity in comparison with phosphonoform acid and its physiologically acceptable salts. In conclusion compounds of the invention have antiviral effects on herpes and influenza viruses. Furthermore compounds of the invention can be bio-transformed to phosphonoformic acid or ionized forms thereof which have strong activities against viral functions and virus multiplication.

Claims

1. A pharmaceutical preparation containing as active ingredient a compound of the formula

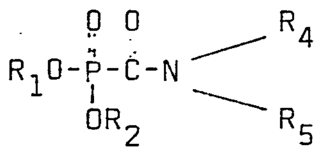

I

wherein $R_1$ and $R_2$, which are the same or different, each is selected from the group consisting of hydrogen, alkyl groups containing 1-6 carbon atoms; cycloalkyl groups containing 3-6 carbon atoms; cycloalkyl-alkyl groups containing 4-6 carbon atoms; phenyl; benzyl, and wherein $R_4$ and $R_5$ are the same or different, and each are selected from the group consisting of hydrogen, alkyl groups containing 1-6 carbon atoms, cycloalkyl groups containing 4-6 carbon atoms, phenyl, and benzyl; or $R_4$ and $R_5$ together with the nitrogen atom may form a pyrrolidine or piperidine ring; or a physiologically acceptable salt or an optical isomer thereof.

2. A pharmaceutical preparation according to claim 1 in dosage unit form.

3. A pharmaceutical preparation according to any of claims 1-2, designed for systemic administration.

4. A compound of the formula

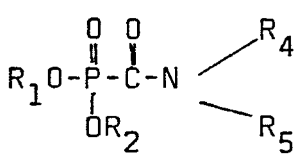

I

wherein $R_1$ and $R_2$, which are the same or different. each is selected from the group consisting of hydrogen, alkyl groups containing 1-6 carbon atoms; cycloalkyl groups containing

3-6 carbon atoms; cycloalkyl-alkyl groups containing 4-6 carbon atoms; phenyl; and benzyl, and wherein $R_4$ and $R_5$ are the same or different, and each are selected from the group consisting of hydrogen, alkyl groups containing 1-6 carbon atoms, cycloalkyl groups containing 4-6 carbon atoms, phenyl, and benzyl; or $R_4$ and $R_5$ together with the nitrogen atom may form a pyrrolidine or piperidine ring; provided that

a) both of $R_1$ and $R_2$ are hydrogen; or
b) $R_1$ is hydrogen and $R_2$ is phenyl, benzyl, cycloalkyl, or cycloalkyl-alkyl; or
c) at least one of the groups $R_1$, $R_2$, $R_4$ and $R_5$ are cycloalkyl or cycloalkyl-alkyl; or
d) at least one of the groups $R_4$ and $R_5$ is benzyl; and physiologically acceptable salts thereof

5. A compound according to claim 4 wherein both of $R_1$ and $R_2$ are hydrogen.

6. A compound according to claim 4 wherein $R_1$, $R_2$, $R_4$ and $R_5$ are combined as follows:

| $R_1$ | $R_2$ | $R_4$ | $R_5$ |
|---|---|---|---|
| H | H | H | $C_2H_5$ |
| H | H | $C_2H_5$ | $C_2H_5$ |
| H | H | H | benzyl |
| $C_2H_5$ | $C_2H_5$ | H | benzyl |
| H | $C_2H_5$ | H | benzyl |

7. Use of a compound as defined in claim 1

a) in the treatment of diseases caused by viruses in animals including man,

b) for the treatment of virus-induced neoplastic diseases in animals including man, by inhibiting the transformation of virusinfected cells,

c) for the treatment of diseases caused by viruses in animals including man, by inhibiting the activity of viral polymerase,

d) for inhibiting the activity of reverse transcriptases of viruses in animals including man,

e) for inhibiting the multiplication of virus, in particular herpesviruses, influenza virus and hepatitis B virus, and retroviruses in animals including man,

f) for inhibiting the growth of virus-transformed cells in animals including man,

g) for the treatment of virus-induced neoplastic diseases in animals including man, by inhibiting the multiplication of tumor viruses,

h) for the treatment of virus-induced neoplastic diseases in animals including man by inhibiting the activity of reverse transcriptase,

i) for the treatment of neoplastic diseases in animals including man.

8. A process for the preparation of novel compounds within the formula

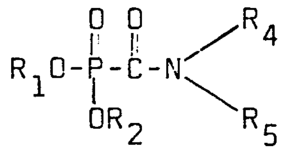

I

wherein $R_1$ and $R_2$, which are the same or different, each is selected from the group consisting of hydrogen, alkyl groups containing 1-6 carbon atoms; cycloalkyl groups containing 3-6 carbon atoms; cycloalkyl-alkyl groups containing 4-6 carbon atoms; phenyl; and benzyl, and wherein $R_4$ and $R_5$ are the same or different, and each are selected from the group consisting of hydrogen, alkyl groups containing 1-6 carbon atoms, cycloalkyl groups containing 4-6 carbon atoms, phenyl, and benzyl; or $R_4$ and $R_5$ together with the nitrogen atom may form a pyrrolidine or piperidine ring; or a physiologically acceptable salt or an optical isomer thereof, by known methods such as

A. Reacting a hydroxycarbonylphosphonic acid triester with ammonia or an amine, according to the formula

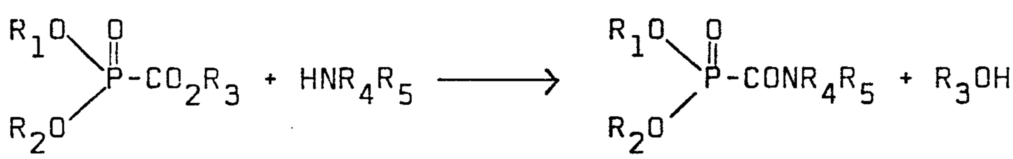

wherein $R_1$, $R_2$, $R_4$ and $R_5$ have the meaning given above; $R_1$ and $R_2$ may be the same or different, and $R_4$ and $R_5$ may be the same or different; and $R_3$ is an aliphatic, cycloaliphatic, araliphatic or aromatic leaving group, such as alkyl groups containing 1-8 carbon atoms, cycloalkyl groups containing 3-8 carbon atoms, cycloalkyl-alkyl groups containing 4-8 carbon atoms, phenyl, and benzyl;

B. Reacting a phosphite triester with carbamoyl compounds according to the formula:

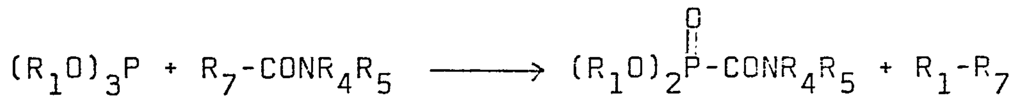

wherein $R_7$ is a leaving group suitable for Arbuzow type reactions, such as Cl, Br, I, sulphonate, carboxylate, alkoxide, and $R_1$, $R_4$, and $R_5$ have the meaning given above;

C. Reacting a phosphite triester with carbamoyl compounds according to the formula:

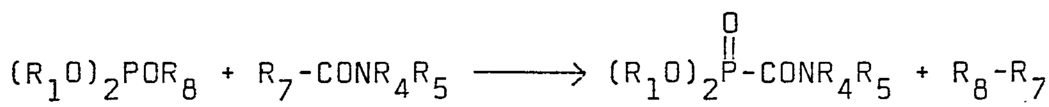

wherein $R_1$, $R_4$, $R_5$ and $R_7$ have the meaning given above; $R_8$ is an alkyl, a cycloalkyl, a cycloalkyl-alkyl, a benzyl, an adamantyl or any phosphite esterifying group suitable for participating in Arbuzow type reactions;

D. Reacting a phosphite triester with carbamoyl compounds according to the formula:

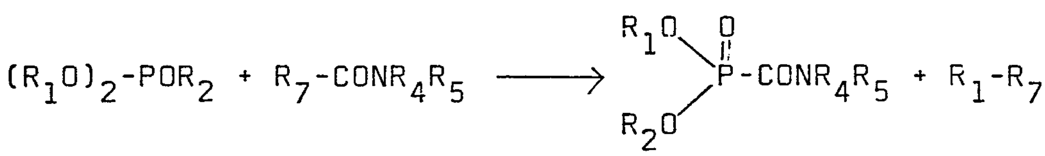

wherein $R_1$, $R_2$, $R_4$, $R_5$ and $R_7$ have the meaning given above, except that $R_1$ must not be phenyl;

E. Reacting a phosphite diester salt with carbamoyl compounds according to the formula:

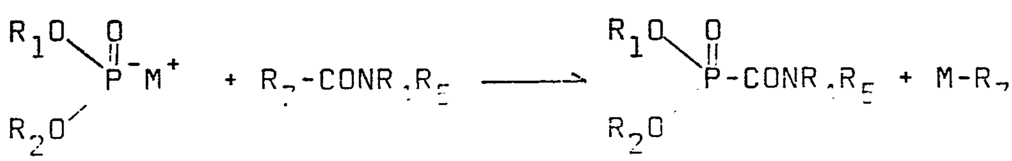

wherein $R_1$, $R_2$, $R_4$, $R_5$ and $R_7$ have the meaning given above, and $R_1$ and $R_2$ may be the same or different; and M is a

cation such as Li Na or K;

F. Reacting phosphite diesters with isocyanates according to the formula:

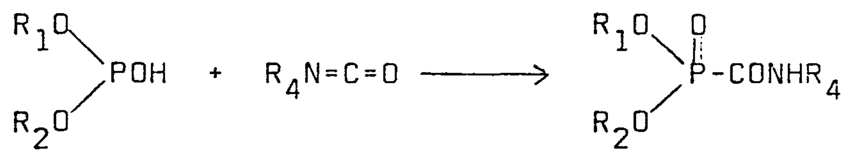

wherein $R_1$, $R_2$ and $R_4$ have the meaning given above and $R_1$ and $R_2$ may be the same or different;

G. Esterification of carbamoylphosphonic acid according to the formula

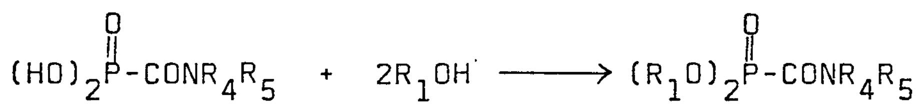

wherein $R_1$, $R_4$ and $R_5$ have the meaning given above;

H. Esterification of carbamoylphosphonic acid monoester according to the formula:

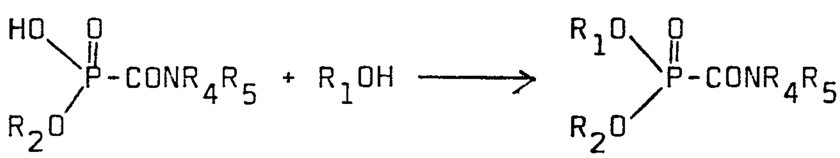

wherein $R_1$, $R_2$, $R_4$ and $R_5$ have the meaning given above;

J. Reacting carbamoylphosphonic acid dihalides according to the formula:

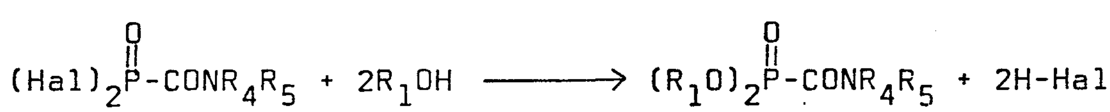

wherein Hal is Cl, Br or I; and $R_1$, $R_4$ and $R_5$ have the meaning given above;

K. Reacting carbamoylphosphonic acid moncester halide according to the formula:

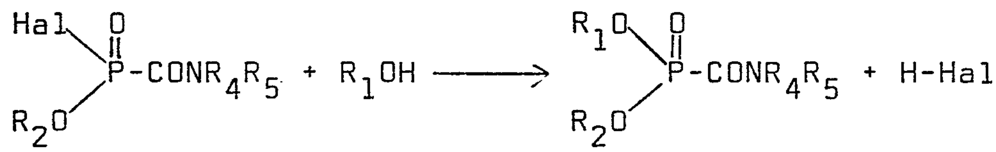

wherein Hal is Cl, Br or I and $R_1$, $R_2$, $R_4$ and $R_5$ have the meaning given above;

L. Hydrolysis of carbamoylphosphonic acid diesters according to the formula:

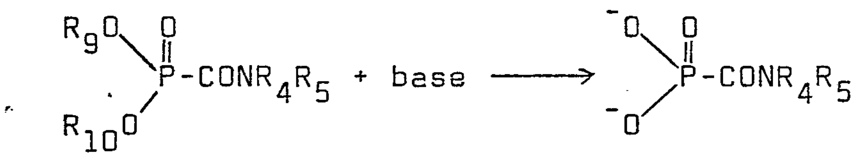


wherein $R_4$ and $R_5$ have the meaning given above; $R_9$ and $R_{10}$ have the meaning given $R_1$ and $R_2$ and $R_9$ and $R_{10}$ may be the same or different;

M. Aqueous hydrolysis of carbamoylphosphonic acid diesters according to the formula

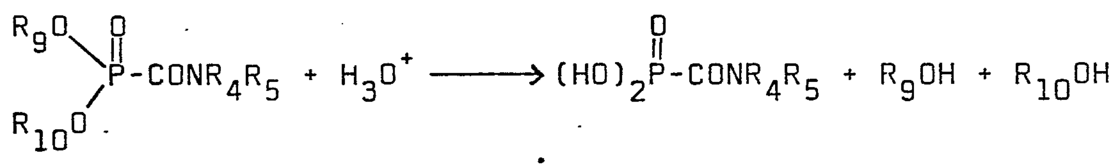

wherein $R_4$, $R_5$, $R_9$ and $R_{10}$ have the meaning given above;

N. Reacting carbamoylphosphonic acid diesters with hydrohalide acids according to the formula

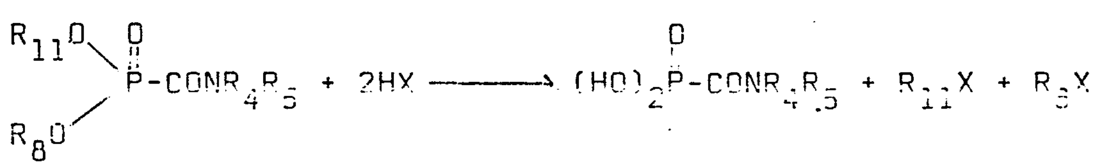

wherein $R_4$, $R_5$ and $R_8$ have the meaning given above; $R_{11}$ has the meaning given $R_8$ and $R_8$ and $R_{11}$ may be the same or different; and X is Cl, Br or I;

O. Aqueous hydrolysis of a carbamoylphosphonic acid containing two silyl esterified phosphonate groups, according to the formula

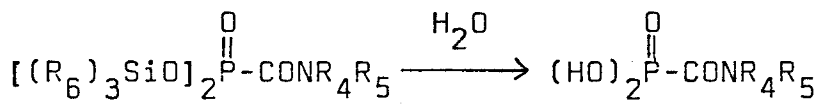


wherein $R_4$ and $R_5$ have the meaning given above, and $R_6$ is an inert organic residue;

P. Hydrogenating dibenzyl carbamoylphosphonates according to the formula

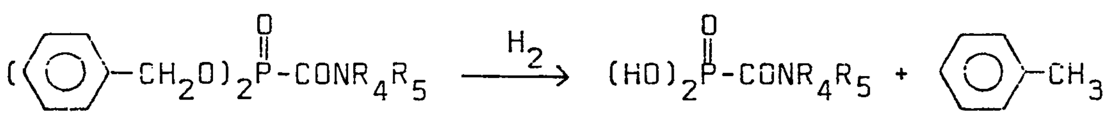


wherein $R_4$ and $R_5$ have the meaning given above;

R. Reacting a carbamoylphosphonic acid diester with an iodide or bromide anion according to the formula

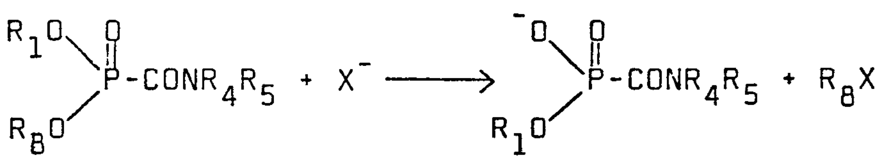

wherein $R_1$, $R_4$, $R_5$ and $R_8$ have the meaning given above, and $X^-$ is $Br^-$ or $I^-$;

S. Hydrolyzing a carbamoylphosphonic acid diester with a base according to the formula

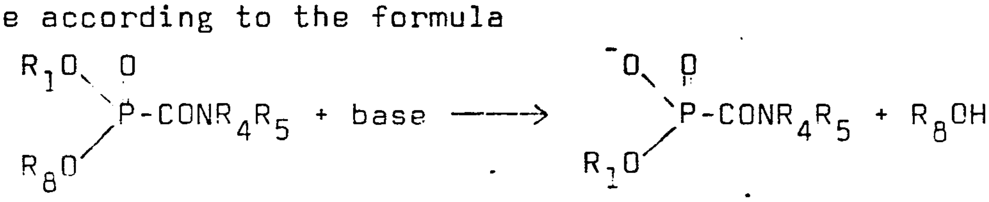

wherein $R_1$, $R_4$, $R_5$ and $R_8$ have the meaning given above;

T. Aqueous hydrolysis of a carbamoylphosphonic acid diester, containing a silylesterified phosphonate group, according to the formula

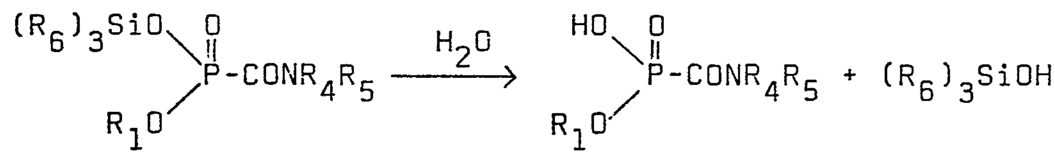


wherein $R_1$, $R_4$ and $R_5$ have the meaning given above and $R_6$ is an inert organic residue;

U. Reacting carbamoylphosphonic acid according to the formula

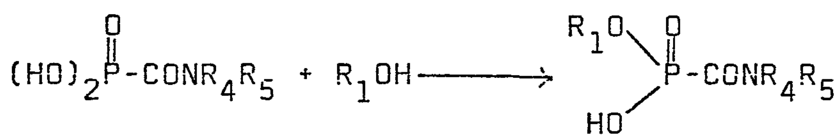

wherein $R_1$, $R_4$ and $R_5$ have the meaning given above.

European Patent Office

# EUROPEAN SEARCH REPORT

0003008

Application number

EP 78 85 0029

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| | US - A - 3 005 010 (GRISLEY)<br>* First column *<br>-- | 4,5,6,8 | C 07 F 9/40<br>A 61 K 31/66 |
| | JOURNAL OF AMERICAN CHEMICAL SOCIETY, vol. 77, pages 3813-15 (1955)<br>* Table I, page 3814 *<br>-- | 4,5,6,8 | |
| AP | FR - A - 2 356 424 (ASTRA)<br>* Claims *<br>---- | 1,2,3,7 | TECHNICAL FIELDS SEARCHED (Int.Cl.²)<br>C 07 F 9/40 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| ✓ | The present search report has been drawn up for all claims | | &: member of the same patent family, corresponding document |

| Place of search | Date of completion of the search | Examiner |
|---|---|---|
| The Hague | 26-03-1979 | MARANDON |

EPO Form 1503.1 06.78